# EUROPÄISCHE PATENTSCHRIFT

(11) **EP 2 694 220 B1**
(45) Veröffentlichungstag und Bekanntmachung des Hinweises auf die Patenterteilung: **06.05.2020**
(21) Anmeldenummer: 12710275.4
(22) Anmeldetag: 23.03.2012
(51) Int. Cl.: A61M 15/00, B05B 11/00

(54) **MEDIZINISCHES GERÄT MIT BEHÄLTER**
MEDICAL DEVICE COMPRISING A CONTAINER
APPAREIL MÉDICAL POURVU D'UN RÉCIPIENT

(30) Priorität: 01.04.2011 EP 11160773
(43) Veröffentlichungstag der Anmeldung: 12.02.2014
(73) Patentinhaber: Boehringer Ingelheim International GmbH, 55216 Ingelheim am Rhein (DE)
(72) Erfinder: HOLAKOVSKY, Holger, 55216 Ingelheim am Rhein (DE); LANGE, Lars, 55216 Ingelheim am Rhein (DE); STEINZEN, Maurice, 55216 Ingelheim am Rhein (DE); WEILAND, Felix, 55216 Ingelheim am Rhein (DE); LIST, Klaus, 64385 Reichelsheim (DE)
(74) Vertreter: Simon, Elke Anna Maria
(86) Internationale Anmeldenummer: PCT/EP2012/055209
(87) Internationale Veröffentlichungsnummer: WO 2012/130757

(56) Entgegenhaltungen:
- EP-A1- 0 679 443
- WO-A1-2009/047021
- GB-A- 2 333 129
- US-B1- 6 685 691

## Beschreibung

Die vorliegende Erfindung betrifft Geräte zum Verabreichen von flüssigen, medizinischen Formulierungen, die fluidische Verbindung dieser Geräte mit beispielsweise treibgasfreien Behältern, welche die jeweilige Flüssigkeit enthalten, und die Abdichtung der Behälter und der fluidischen Verbindung zwischen Behälter und Gerät nach außen. Insbesondere bezieht sich die Erfindung auf kleinere portable Geräte wie beispielsweise von Hand betriebene Zerstäuber oder Injektoren, wie sie für die Inhalation oder Injektion von flüssigen Arzneimittelformulierungen verwendet werden.

Aus dem Stand der Technik ist eine Vielzahl an medizinischen Geräten und Zerstäubern bekannt, die mit einer Flüssigkeit betrieben werden. Bei den meisten ist die Flüssigkeit in Vorratsgefäßen oder Behältern abgefüllt, die mehrere Einheiten der Flüssigkeit zur Verabreichung mit dem Gerät enthalten. Zur Entnahme der Flüssigkeit bzw. der Einheiten in Form von abgemessenen Teilmengen aus dem Behälter wird eine fluidische Verbindung zwischen Gerät und Behälter hergestellt, je nach Anwendung dauerhaft oder unterbrechbar. Die fluidische Verbindung wird entweder durch Entnahmeleitungen wie Kanülen oder Schläuche, die in den Behälter eingeführt werden, oder durch das Freilegen von Öffnungen im Behälter und deren Anschluss an zugehörige Kanäle im Geräteinnern hergestellt. Zu letztere Gruppe zählen auch Systeme wie beispielsweise die Treibgas-enthaltenden Metered Dose Inhaler (MDIs), in denen eine Vorkammer oder Dosierkammer in den Behälter integriert ist und über einen Umschaltmechanismus - z.B. in Form eines beweglichen Stößels mit entsprechenden Durchführungen - die Vorkammer entweder mit dem Flüssigkeitsvorrat oder mit dem Gerät verbunden wird.
Allen diesen Systemen gemeinsam ist die Notwendigkeit, die Verbindungsstelle zwischen Behälter und Gerät - sei sie nun statisch oder dynamisch bewegt - nach außen hin abzudichten, so dass keine Flüssigkeit aus dem System außer über den vorgesehenen Ausbringungsweg entweicht.
Die Forderungen an die Abdichtung der Verbindung zwischen Gerät und Behälter hängen insbesondere bei Handgeräten wie Zerstäubern von der zu verabreichenden Arzneimittelformulierung und ihrer Konzentration, dem verwendeten Lösemittel, oder von klimatischen Bedingungen am Einsatzort ab und können somit stark variieren. Die Schnittstelle von Behälter zu Gerät muss gegen alle Substanzen aus der Arzneimittelformulierung dicht und materialbeständig sein. Die Formulierung kann nicht nur flüssige und feste sondern auch gasförmige Bestandteile enthalten, wobei Gasdichtigkeit in der Regel eine höhere Forderung als Flüssigkeitsdichtigkeit darstellt. Einige Arzneimittelformulierungen enthalten leicht flüchtige Substanzen wie beispielsweise das oft als Lösemittel eingesetzte Ethanol. Beim separaten Entweichen einer flüchtigen Substanz aus dem Behälter kann sich die Konzentration der Formulierung ändern. Je nach Zusammensetzung der Formulierung kann dadurch die Konzentration eines Wirkstoffs in der Lösung erhöht werden oder ein gelöster Stoff kann auskristallisieren. Solche Substanzen entweichen in erster Linie in gasförmiger Form: Diese Substanzen, die von Natur aus einen erhöhten Dampfdruck haben, wechseln insbesondere bei klimatischen Veränderungen auch innerhalb des Behälters schnell teilweise in die Gasphase über. Unter Umständen können bereits kleine Temperaturerhöhungen oder Drucksenkungen in der Umgebung des Behälters zu einer verstärkten Gasbildung im Behälter führen, und dieses Gas kann teilweise durch eine in erster Linie auf Flüssigkeitsdichtigkeit ausgelegte Dichtung entweichen.

Ein mechanischer, miniaturisierter Hochdruckzerstäuber, mit dem flüssige Arzneimittelformulierungen zur Inhalation aus einem mehrere Einheiten der Formulierung enthaltenden Behälter zerstäubt werden und in dessen Inneren der Fluidweg statisch und dynamisch abgedichtet ist, ist aus der WO97/12687A1 und WO2009/047173A2 bekannt. Mit diesem Zerstäuber wird eine flüssige Arzneimittelformulierung aus einem in den Zerstäuber eingesetzten starren Behälter mit kollabierbarem Innenbeutel, wie in der WO00/49988A2 offenbart, mittels einer durch ein Schraub-Schub-Getriebe angetriebenen Kolbenpumpe aus dem Innenbeutel gefördert und mittels eines federgetriebenen Druckpumpe durch eine mikrostrukturierte Düse zu einem lungengängigen Aerosol zerstäubt. Details möglicher Mikrostrukturen für die in den Zerstäuber eingesetzte Austragsdüse werden in den Schriften WO94/07607A1, WO99/16530A1, WO2005/000476A1 und WO2007/101557A2 offenbart. In der WO2004/053362A1 wird ein in solchen Zerstäubern einsetzbares Kolbenpumpensystem beschrieben, in dem über die axiale Bewegung eines Hohlkolbens mit Rückschlagventil Flüssigkeit in vorbestimmter Menge aus dem Vorratsgefäß in einen Pumpenzylinder eingesaugt und aus diesem über einen Flüssigkeitsauslass ausgetrieben wird. Hohlkolben und Kammer werden durch eine elastomere O-Ring-Dichtung in der Führungsröhre des Hohlkolbens nahe dessen Eintritt in den Pumpenzylinder abgedichtet; die geometrische Einbausituation dieser Dichtung ist in der WO2007/051536A1 weiterführend beschrieben.

In der WO00/49988A2 wird eine mit einem Stopfen verschlossene, mit Flüssigkeit gefüllte Kartusche gezeigt, die mit dem Anschlussteil eines Entnahmegeräts bzw. eines Zerstäubers verbunden wird. Der Stopfen weist einen Eintauchstutzen mit einer trichterförmigen zentrierten Führung für Anbindung eines zum Anschlussteil gehörenden, rohrförmig abgebildeten Entnahmestutzens auf. Der Stopfen bildet eine Presspassung mit dem eingeführten Entnahmestutzen (siehe WO96/06011A1 für Varianten dieses Stopfens in Form einer Behälter-Verschlusskappe). Kartusche und Anschlussteil werden über eine Steckverbindung verbunden, bei der mehrere Schnapphaken am Anschlussteil in eine umlaufende Rille im oberen Bereich der Kartusche eingreifen. Vor der Anbindung an das Entnahmegerät ist die Kartusche, bzw. das obere offene Ende des Eintauchstutzens mit einer Siegelfolie versiegelt, während das dem Innenraum der Kartusche zugewandte Ende des Eintauchstutzens mit einer Membran versehen ist, die beim Einführen des Entnahmestutzens durchstochen oder aufgeklappt wird.

In der US 6685691 B1 wird ein Behälter mit Folienbeuteln als Primärverpackung für Flüssigkeiten gezeigt. Der Behälter umfasst einen kollabierbaren Folienbeutel (beispielsweise aus einer Kompositfolie, die eine Metallfolie beinhaltet), an dem ein stabiler Flansch angeordnet ist, welcher an ein Entnahmelement anschließbar ist. Der ein- oder mehrteilige Flansch ist mit einem Führungskanal für das Entnahmeelement ausgestattet, das es beispielsweise in einer Presspassung umschließt oder gegen das eine Ring-Dichtung im Flansch abdichtet.

WO2006/087516A1 zeigt eine Dichtungsanordnung zur Anbindung des Ventilstammes eines druckbeaufschlagten Behälters an einen Zerstäuber bzw. an eine Schaltvorrichtung für einen Zerstäuber. Diese Dichtungsanordnung beinhaltet einen ersten Dichtungsteil, der direkt auf dem Auslass des Behälters, d.h. auf der Stirnseite des Ventilstammes aufliegt, und einem zweiten davon beabstandeten Dichtungsteil, der die Seitenwand des Ventilstammes abdichtet. Der erste Dichtungsteil ist eine Flachdichtung mit Durchgangsloch und der zweite eine O-Ring-Dichtung. Die beiden Dichtungen sind bzgl. ihrer Dichtfunktion redundant zueinander. Beide werden inklusive eines Abstandhalters von einer festen Kappe zusammengehalten und bilden so eine mehrteilige Dichtanordnung.

Der vorliegenden Erfindung liegt die Aufgabe zugrunde, ein gegenüber dem Stand der Technik verbessertes Gerät - insbesondere ein Handgerät wie einen Zerstäuber oder Injektor - zum Verabreichen von medizinischen Formulierungen aus einem Behälter anzugeben, bei dem die Verbindungsstelle von Behälter und Gerät entsprechend der verwendeten Formulierung flüssigkeitsdicht und gasdicht abgedichtet ist. Insbesondere soll das Dichtsystem keine Permeabilität gegenüber den flüssigen und gasförmigen Substanzen der Formulierung aufweisen bzw. es soll keine Diffusionsleckagen zulassen, insbesondere wenn die Formulierungen Substanzen mit hohem Dampfdruck wie z.B. Ethanol enthalten. Das Gerät mit Dichtsystem an der Verbindungsstelle von Behälter und Gerät soll insbesondere für das Bereitstellen von abgemessenen Flüssigkeitsmengen geeignet sein. Die mit diesen Dichtsystemen ausgestatteten Geräte sollen hierbei möglichst unabhängig von der späteren Verwendung, d.h. insbesondere unabhängig von klimatischen Bedingungen und insbesondere unabhängig von klimatischen Schwankungen oder von der jeweiligen für den Anwender unter Umständen individuell festgelegten Benutzung oder Therapie sein. Je nach Therapie kann die pro Gerät vorgesehene Anzahl an Betätigungen pro Tag abhängig von Wirkstoffformulierung und Dosierung von Gerät zu Gerät variieren. Des weiteren soll das Gerät mit Dichtsystem für die Massenfertigung geeignet sein. Insbesondere soll das Dichtsystem hinsichtlich Anzahl und Art seiner Bauteile möglichst preisgünstig sein und es soll zuverlässig ohne Beschädigungen in Massenfertigung montierbar sein.

Die genannte Aufgabe wird erfindungsgemäß gelöst durch ein Gerät zum Verabreichen einer flüssigen medizinischen Formulierung, die sich in einem in das Gerät eingesetzten Behälter befindet. Der Behälter ist innerhalb des Geräts auf ein steifes Rohr aufgeschoben ist, das mit einer Halterung verbunden ist, die den Behälter im Gerät aufnimmt, zum Beispiel durch Einklemmen. Der Behälter weist eine Einführstelle auf, an der eine erste Dichtung in Form einer Passdichtung zwischen Behälter und einem Abschnitt des steifen Rohres vorliegt, das der Flüssigkeitsentnahme aus dem Behälter dient. Zwischen der Halterung und dem Behälter ist eine zweite Dichtung vorhanden, die den Raum zwischen der ersten Dichtung, dem Behälter und dem Rohr gegen den Austritt von Flüssigkeit und Gasen und/oder gegen das Eindringen von Gasen abdichtet.
Die zweite Dichtung dichtet die fluidische Verbindung zwischen Gerät und Behälter gegenüber der Umgebung zusätzlich ab. Durch die beiden aufeinander folgenden Dichtungen wird das nicht gewollte Austreten von Flüssigkeit und Gas aus dem Behälter und/oder das Eindringen von Gas in den Behälter besser verhindert als durch die erste Dichtung allein.

Vorteilhafte Weiterbildungen werden im Folgenden und im Detail anhand der Figuren beschrieben.

Ein Merkmal der vorliegenden Erfindung ist, dass die erste Dichtung im wesentlichen dicht gegenüber den flüssigen Bestandteilen der medizinischen Formulierung im Behälter und die zweite Dichtung im wesentlichen dicht gegenüber Gasen ist. Hierdurch werden die Forderungen an die Dichtigkeit des Systems insgesamt in unterschiedliche Forderungen an zwei voneinander separate Dichtungen aufgeteilt. Das bietet den Vorteil, dass die einzelnen Forderungen gezielt erfüllt werden können, ohne unter dem Zwang zu stehen, eine einzige u.U. teure oder technisch aufwendige oder hinsichtlich von Teilaspekten mangelhafte Lösung zu benutzen. Auf diese Weise kann beispielsweise eine erste behälterseitige Dichtung in erster Linie auf die Rückhaltung der im Behälter befindlichen Flüssigkeit unter voller Berücksichtigung der Forderungen an die Materialverträglichkeiten von Flüssigkeit und Dichtungsmaterial ausgelegt sein. Die Forderung der Gasdichtigkeit der fluidischen Verbindung des Geräts mit dem Behälter kann mittels der zweiten Dichtung gezielt gelöst werden. Dies bedeutet u.a., dass für diese zweite Dichtung, die auch als Gasphasendichtung bezeichnet werden kann, beispielsweise das Material in erster Linie unter dem Aspekt der Gasdurchlässigkeit gewählt werden kann, ohne dass die hierbei verwendeten Materialien zwangsläufig chemisch verträglich zur Flüssigkeit im Behälter sein müssen. Dies ist insbesondere relevant für Systeme, in denen der Behälter leicht flüchtige Substanzen wie beispielsweise Ethanol enthält. Leicht flüchtige Substanzen zeigen sowohl aufgrund ihres Dampfdrucks eine hohe Diffusion durch feinste Kanäle, als auch bei andauerndem fluidischen Kontakt eine signifikante Diffusion durch eine Vielzahl von Kunststoffmaterialien.

Ein weiteres Merkmal der vorliegenden Erfindung ist, dass während die erste Dichtung durch eine Passdichtung oder Presspassung zwischen dem Behälter - bevorzugt einem Teilbereich eines in eine Behälterkappe integriertem Einführtrichter - und dem der Flüssigkeitsentnahme dienendem Rohr gebildet wird, die zweite Dichtung durch nur ein zusätzliches Bauteil oder eine zusätzliche Dichtungsschicht zwischen dem Behälter bzw. der Behälterkappe und der Halterung des Behälters im Gerät gebildet wird. Die Dichtungsschicht, deren Material vorzugsweise weicher als das von Behälterkappe und Halterung ist, kann z.B. durch Anspritzen entweder auf der dem Behälter zugewandten Seite der Halterung oder am Innenrand der Einführstelle am Behälter bzw. im oberen Bereich der Behälterkappe angebracht sein. Insbesondere wirken beide Dichtungen - erste und zweite - durch direkten Kontakt zur Behälterkappe.
Eine durch ein zusätzliches Bauteil gebildete Dichtung wird vorzugsweise am Gerät angebracht. Sie besteht aus nur einem elastomeren Bauteil, das beim Andocken des Behälters zwischen Behälterkappe und Gerät verpresst wird. Diese geräteseitige Dichtung kann eine kappenartige oder becherartige oder hülsenartige oder konusartige Form mit Durchlassöffnung für das Rohr aufweisen oder eine O-Ring-Dichtung, Flachdichtung oder Ringdichtung sein.
Alternativ zum Einsatz einer elastomeren Dichtung können beide Dichtungen durch den direkten Kontakt von Behälterkappe zu harten Bauteilen des Geräts gebildet werden. Insbesondere können durch Presspassungen zwischen der Behälterkappe und dem steifen, der fluidischen Verbindung zwischen Behälter und Gerät dienenden Rohr einerseits und einer die Behälteraufnahme bildenden Halterung am Gerät andererseits gebildet werden.
Durch eine solche Maßnahme wird eine zusätzliche Abdichtung der Verbindungsstelle von Behälter zu Gerät mit keinem oder nur einem zusätzlichen Bauteil erzielt. Dieses Dichtsystem ist kostengünstig und für die Massenfertigung geeignet. Die doppelte Abdichtung hat zusätzlich den Vorteil, dass sich gelegentliche Undichtigkeiten nicht auf die Dichtigkeit des Systems insgesamt auswirken können. Derartige Undichtigkeiten können durch sporadisch auftretende Unebenheiten auf einer zu dem Dichtungssystem gehörenden harten Oberfläche, wie beispielsweise der Oberfläche des Rohrs im Bereich der Presspassung mit der Behälterkappe, verursacht werden, Eine zweite Dichtung fängt eine eventuelle Leckage an der Stelle der ersten auf. Hierdurch können unter Umständen Forderungen an Produktionsprozess und ggf. Produktionskosten gesenkt werden.

Bei den hier dargestellten Geräten zum Verabreichen von medizinischen Formulierungen werden bevorzugt Handgeräte wie Zerstäuber oder Injektoren betrachtet, mit denen Flüssigkeiten in vorbestimmten Volumina oder definierten Mengen zerstäubt oder eingespritzt werden.
Der Begriff "Flüssigkeit" umfasst neben reinen Flüssigkeiten und Lösungen zusätzlich Dispersionen, Suspensionen, Suslutionen (Mischungen aus Lösungen und Suspensionen) oder dergleichen. Unter dem Begriff "medizinische Formulierung" oder "Arzneimittelformulierung" sind bei der vorliegenden Erfindung über Medikamente hinaus auch Therapeutica oder dergleichen, insbesondere also jede Art von Mitteln zur Inhalation oder sonstigen Anwendung bei Mensch und Tier zu verstehen.

Die einzelnen Merkmale der vorliegenden Erfindung können unabhängig voneinander genutzt oder miteinander kombiniert werden.
Weitere Vorteile, Merkmale, Eigenschaften und Aspekte der vorliegenden Erfindung ergeben sich aus der folgenden Beschreibung bevorzugter Ausführungsformen anhand der Zeichnungen. Die Zeichnungen zeigen in:
- Fig. 1: einen schematischen Längsschnitt eines Zerstäubers im "ungespannten" Zustand,
- Fig. 2: einen schematischen, um 90° gegenüber Fig.1 gedrehten Längsschnitt des Zerstäubers aus Fig. 1 im "gespannten" Zustand,
- Fig. 3: einen schematischen Längsschnitt durch die Zerstäuber-Bauteile Düse, Filter, Pumpenkammer, Hohlkolben und Halterung für den nicht dargestellten Behälter.
- Fig. 4: einen schematischen Längsschnitt durch die Anbindungsstelle eines Behälters an eine Förderpumpe mit einer erfindungsgemäßen Abdichtung
- Fig. 5: einen schematischen Längsschnitt durch die Anbindungsstelle eines Behälters an eine Förderpumpe gemäß einer ersten Alternative zur erfindungsgemäßen Abdichtung
- Fig. 6: einen schematischen Längsschnitt durch die Anbindungsstelle eines Behälters an eine Förderpumpe gemäß einer zweiten Alternative zur erfindungsgemäßen Abdichtung
- Fig. 7: einen schematischen Längsschnitt durch die Anbindungsstelle eines Behälters an eine Förderpumpe gemäß einer dritten Alternative zur erfindungsgemäßen Abdichtung
- Fig. 8: einen schematischen Längsschnitt durch die Anbindungsstelle eines Behälters an eine Förderpumpe gemäß einer vierten Alternative zur erfindungsgemäßen Abdichtung
- Fig. 9: einen schematischen Längsschnitt durch die Anbindungsstelle eines Behälters an eine Förderpumpe gemäß einer fünften Alternative zur erfindungsgemäßen Abdichtung

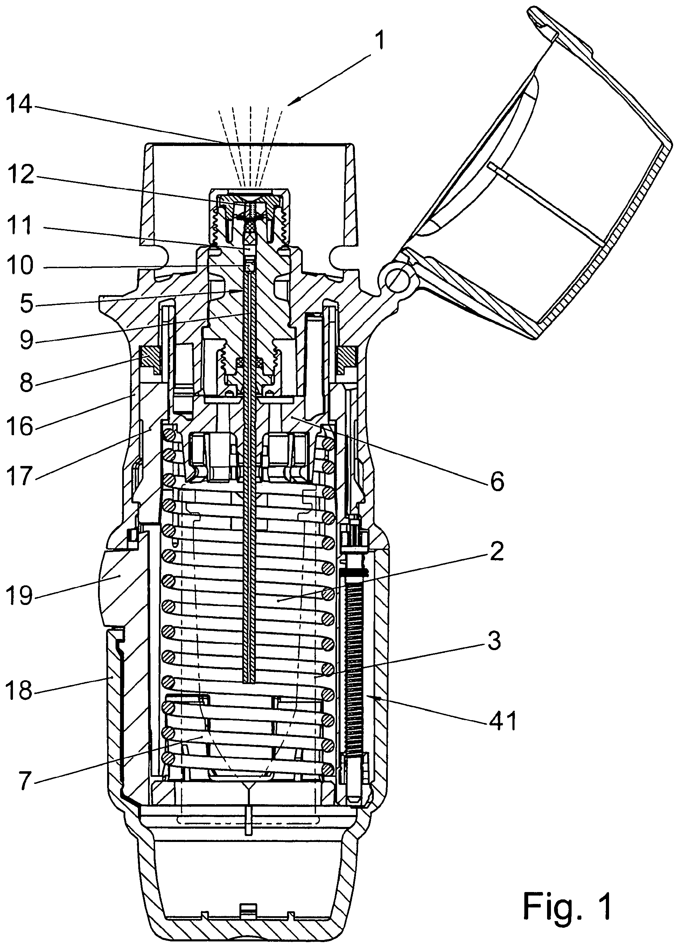

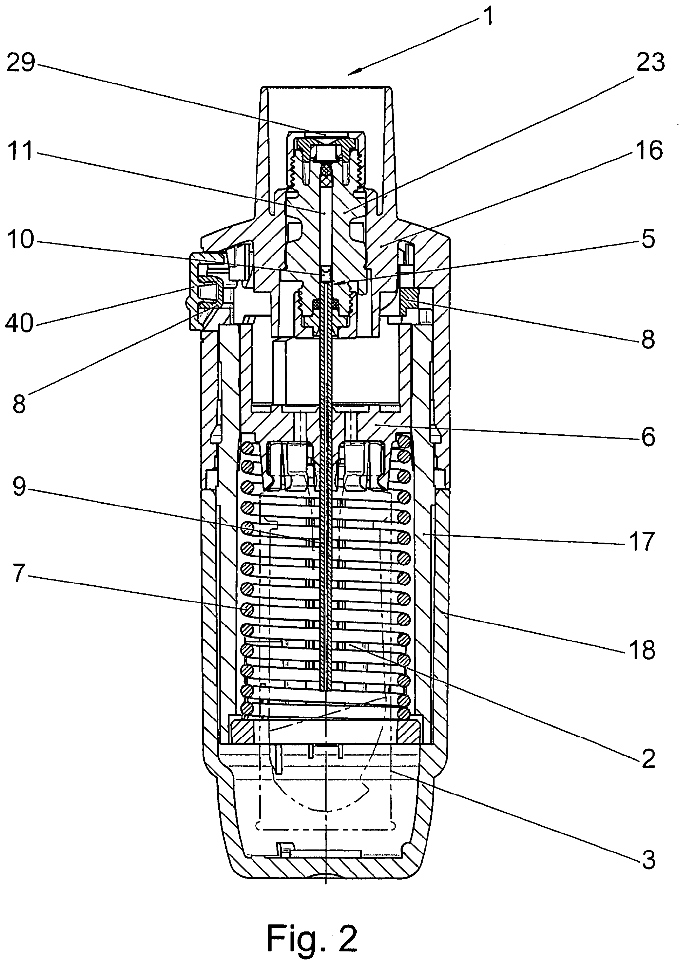

In den Figuren werden für gleiche oder ähnliche Teile dieselben Bezugszeichen verwendet, wobei entsprechende oder vergleichbare Eigenschaften und Vorteile erreicht werden, auch aus Fig. 1 und 2 handelt es sich um einen treibgasfreien Zerstäuber (1), der mittels einer rein mechanischen Hochdruckpumpe pro Betätigungszyklus die jeweilig vorbestimmte Menge einer Flüssigkeit (2) bzw. einer flüssigen medizinischen Formulierung als vorzugsweise lungengängiges bzw. inhalierfähiges Aerosol (14) aus der Düse (12) ausgibt. Dieses Aerosol (14) mit Tröpfchen mit aerodynamischen Durchmessern von vorzugsweise 3 bis 10 Mikrometern kann von einem nicht dargestellten Benutzer eingeatmet werden. Tauscht man die der Zerstäubung dienende Düse (12) dieses Geräts gegen den Kopf eines Flüssigkeitsspenders oder eine Injektionsdüse bzw. eine Kanüle oder sonstige Einspritzvorrichtung, so bleiben alle von der Düse unabhängigen Funktionsprinzipien unverändert. Die im Nachfolgenden geschilderten Zusammenhänge haben ihre Gültigkeit analog für Injektoren oder andere Flüssigkeits-ausbringende Systeme, selbst wenn der Einfachheit halber nur Zerstäuber genannt werden.
Beim Betrieb des Zerstäubers wird unterschieden zwischen dem so genannten "ungespannten" Zustand mit unbefülltem Dosiervolumen in der Druckkammer (11) (Fig. 1) und dem "gespannten" Zustand mit befüllter Druckkammer (11) (Fig. 2). Die Bezeichnungen "ungespannt" und "gespannt" beziehen sich gleichzeitig auf den Zustand der im Zerstäuber eingebauten Antriebsfeder (7).
Beim so genannten "Spannen" des Zerstäubers (1) wird sein Gehäuseoberteil (16) relativ zum Gehäuseinnenteil (17) und Gehäuseunterteil (18) um einen festen Drehwinkel z.B. 180° gedreht. Mittels eines innen angeordneten Schraub-Schub-Getriebes wird durch die Relativdrehung eine Kolbenpumpe angetrieben, so dass eine vorbestimmte, ggf. einstellbare Menge der Flüssigkeit (2) aus dem Behälter (3) in die Druckkammer gefördert und gleichzeitig die auf den Hohlkolben (9) wirkende Antriebsfeder (7) gespannt wird. Der Endzustand des Spannvorgangs ist in Fig. 2 dargestellt. Beim Auslösen des Zerstäubers (1) durch Betätigung des Sperrrings (8) über die Taste (40) wird die in der Antriebsfeder (7) gespeicherte Energie freigesetzt. Der zuvor zur Flüssigkeitsförderung benutzte Hohlkolben (9), der gleichzeitig Teil der Hochdruckpumpe des Geräts ist, drückt nun mit geschlossenem Rückschlagventil (10) in die Druckkammer (11), so dass die durch die Hubbewegung des Hohlkolbens (9) vorbestimmte Flüssigkeitsmenge von dort durch die Düse (12) ausgebracht wird. Das Gerät befindet sich nun wieder im entspannten Zustand (Fig. 1).

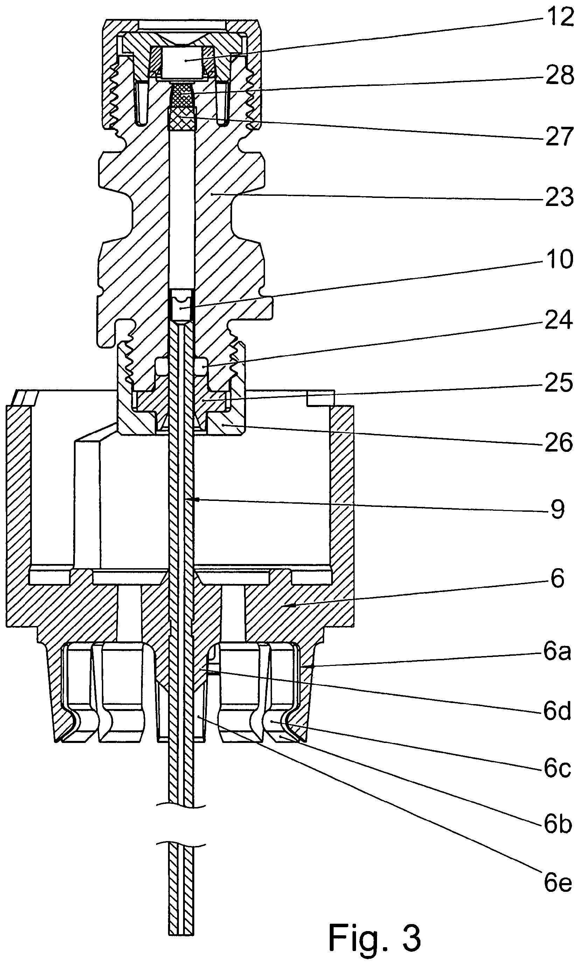

Fig. 3 zeigt in schematischer Darstellung den Druckerzeuger (5) des Zerstäubers (1), der in der dargestellten Ausführungsform sowohl für die Zerstäubung von wässrigen flüssigen Formulierungen als auch insbesondere für die Zerstäubung von Formulierungen geeignet ist, die eine Substanz mit hohem Dampfdruck oder insbesondere eine alkoholische Verbindung z.B. als Lösemittel enthalten. In die Druckkammer (11) taucht behälterseitig der zum Kolbenpumpensystem gehörende Hohlkolben (9) ein. Der Hohlkolben (9) ist zugleich das Verbindungselement zwischen Druckkammer (11) und dem Inneren des Behälters (3). Der Hohlkolben (9) oder ein ähnliches steifes Rohr oder rohrförmiges Bauteil wie eine Kapillare oder Kanüle stellt somit eingeführt in den Behälter (3) die fluidischen Verbindung zwischen Gerät und Behälter (3) her. Wird der Hohlkolben (9) beim Spannvorgang der Antriebsfeder (7) teilweise aus der Druckkammer (11) herausgezogen, entsteht dort ein Unterdruck, durch den über das in dieser Position offene Rückschlagventil (10) im Hohlkolben (9) Flüssigkeit (2) aus dem Behälter (3) in die Druckkammer (11) angesaugt wird. Schiebt sich der Hohlkolben (9) beim Auslösen des Zerstäubers (1) in die Druckkammer (11) hinein, ist das Rückschlagventil (10) durch Anliegen seiner Dichtflächen am Sitz im Hohlkolben geschlossen, und die Flüssigkeit in der Druckkammer (11) wird unter Druck durch ein Filtersystem und die Düse (12) ausgetrieben. Hohlkolben (9) und Druckkammer (11) werden nach außen durch eine elastomere Dichtung (24) abgedichtet, die insbesondere die Form eines O-Rings hat und sich in der Führungsröhre des Kolbens nahe dessen Eintritt in die Druckkammer (11) bzw. der Dosierkammer des Zerstäubers (1) befindet. Da diese Dichtung (24) einen Bauraum gegenüber einem bewegten Teil - dem Hohlkolben (9) - abdichtet, kann man sie als dynamische Dichtung bezeichnen. Somit wird die Hochdruckpumpe durch eine von der Anbindung des Hohlkolbens (9) an den Behälter (3) separate, insbesondere dynamische Dichtung gegenüber dem Hohlkolben (9) abgedichtet. Im Darstellungsbeispiel wird die Dichtung (24) mittels eines Stützrings (25) verpresst, der von einer Überwurfmutter (26) in Position gehalten wird. Die geometrische Einbauposition der Dichtung (24) entspricht beispielsweise der in der WO2007/051536A1 beschriebenen.
Im Flüssigkeitsauslassbereich der Druckkammer (11) befindet sich ein Filtersystem (27, 28), das in Flussrichtung vor der vorzugsweise mikrostrukturierten Düse (12) liegt und diese gegen die Ablagerung von Partikeln schützt. Über die Kombination verschiedenartiger Filter (27, 28) und Filtertechniken wird eine hohe Abscheidung erzielt. Im Falle des Ausführungsbeispiels wird die Düse (12) bevorzugt von einem mikrostrukturierten Bauteil aus einem Glas-Silizium-Verbund gebildet, das selbst bereits einen als Strömungsfilter ausgelegten Feinstfilter vor den eigentlichen Düsenkanälen enthält. Die Zerstäubung der Flüssigkeit durch diese Düsenkanäle beruht vorzugsweise auf der Impaktion von zwei mikroskopischen Flüssigkeitsstrahlen mit hoher Geschwindigkeit aus nur wenigen Mikrometern durchmessenden Düsenkanälen.

Das Zentralteil (23) bildet die seitliche Begrenzung der Druckkammer (11), den Flüssigkeitseinlass in Form der Durchführung für den Flüssigkeits-führenden Hohlkolben (9), den Einbauraum für die gegen den Hohlkolben (9) abdichtende Dichtung (24) und die fluidische Anbindung zur Düsenbaugruppe (29), welche die Düse (12) und diverse zugehörige Halterungs- oder Dichtungsbauteile enthält. In der gezeigten Ausführungsform mit kreiszylindrischer Druckkammer (11) nimmt das Zentralteil (23) in einer zentralen Bohrung ein oder mehrere Filterbauteile im Anschluss an die Druckkammer (11) auf. Im Beispiel sind die Filterbauteile ein Vorfilter (27), vorzugsweise aus einem Kunststoff, und ein Feinfilter (28), vorzugsweise aus Metall. Weiter stromabwärts schließt sich das beschriebene mikrostrukturierte Bauteil an, das Feinstfilter und Düsenkanäle enthält.

Im Darstellungsbeispiel weist der Zerstäuber (1) bzw. sein Druckerzeuger (5) eine Halterung (6) für den Behälter (3) auf. Diese Halterung (6) ist unlösbar mit dem Hohlkolben (9) verbunden - vorzugsweise angespritzt, beispielsweise auch verklebt oder verschnappt. Beim axialen Spannen der Antriebsfeder (7) wird die Halterung (6) mit dem Behälter (3) und dem Hohlkolben (9) bei den Darstellungen nach unten bewegt. Der Behälter (3) wird über die Halterung (6) insbesondere klemmend oder rastend so in dem Zerstäuber (1) fixiert, dass der Hohlkolben (9) in den Fluidraum des Behälters (3) eintaucht und/oder fluidisch mit der Flüssigkeit (2) im Behälter (3) verbunden wird und die Flüssigkeit über den Hohlkolben angesaugt wird. Hohlkolben (9) und Behälter (3) werden somit beim Betrieb des Zerstäubers (1) nach Verbindung des Behälters (3) mit der Halterung (6) nicht mehr zueinander bewegt, d.h. nach Andocken des Behälters (3) an das Gerät gibt es keine Relativbewegung der an der Abdichtung der Verbindungsstelle von Gerät zu Behälter beteiligten Bauteile zueinander. Die Dichtungen zwischen Gerät und Behälter (3) bzw. Behälterkappe (31) sind somit statisch. Dies hat den Vorteil, dass das Dichtsystem, durch die der Flüssigkeitsvorrat selbst vor Leckagen und Diffusion geschützt wird, keinerlei Reibungsbeanspruchung unterliegt und es somit nicht zum Verschleiß der Dichtungen kommen kann. Vorzugsweise bilden Behälter (3) und Halterung (6) eine Steckverbindung, bei der insbesondere mehrere Schnapphaken (6a) der Halterung (6) in eine umlaufende Kontur im oberen Bereich des Behälters (3) eingreifen. Diese Kontur kann beispielsweise eine umlaufende Rille oder wie im Ausführungsbeispiel der untere Kragenrand einer den Behälter (3) verschließenden Behälterkappe (31) sein. In den hier gezeigten Ausführungsformen weist die Halterung 4 bis 12, vorzugsweise 6 oder 12 Schnapphaken oder Rippen auf. Wird der Behälter (3) mit seiner Behälterkappe (31) voran entlang des Hohlkolbens (9) in die Halterung (6) geschoben, so trifft die Behälterkappe (31) zuerst auf die Einführschrägen (6b) an den Schnapphaken (6a). Durch die nach innen geneigten Einführschrägen (6b) werden die Schnapphaken (6a) durch die Behälterkappe (31) nach außen gespreizt, bis die Behälterkappe (31) an den nach innen gerichteten Wülsten (6c) der Schnapphaken (6a) vorbei gleiten kann. Sobald die untere Außenkante der Behälterkappe (31) die Wülste (6c) passiert hat, federn die Schnapphaken (6a) wieder nach innen zurück, so dass die Wülste (6c) den Behälter (3) am unteren Rand der Behälterkappe (31) festhalten. Die Halterung (6) kann bei Bedarf derart ausgebildet sein, dass der Behälter (3) ausgetauscht werden kann. Diese Austauschbarkeit wird über die Federeigenschaften der Schnapphaken (6a) erreicht. Länge, Breite und vor allem Dicke und Material der Halterung (6) werden entsprechend gewählt. Die Halterung (6) besteht vorzugsweise aus einem Kunststoff aus der Gruppe der Thermoplaste wie beispielsweise PPO (Polyphenylenoxid) bzw. PPE (Polyphenylenether) oder PBT (Polybutylenterephthalat). Die Geometrie der Wülste (6c) und der Anteil der Wülste (6c) am inneren Umfangskreis der Halterung (6) werden angepasst. Zur Feineinstellung der notwendigen Kräfte zum Einsetzen oder Entnehmen des Behälters (3) in die oder aus der Halterung (6) ist es zweckdienlich, nur einen Teil der Rippen an der Halterung mit Wülsten (6c) zur Bildung von Schnapphaken (6a) auszustatten. So weist z.B. die Halterung (6) der in Fig. 3 gezeigten Ausführungsform insgesamt 3 Rippen ohne Wulst (6c) auf. Diese Rippen ohne Wulst (6c) dienen lediglich der radialen Führung des in die Halterung (6) eingeführten Behälters (3).

Die Austauschbarkeit des Behälters wird nicht nur durch die Eigenschaften der Halterung (6) sondern auch durch seine Zugänglichkeit bestimmt: Im Darstellungsbeispiel aus Fig. 1 und Fig. 2 ist ein Zerstäuber gezeigt, dessen kappenartiges Gehäuseunterteil (18) den freien Endbereich des in den Zerstäuber eingesetzten Behälters (3) umhüllt. Das Gehäuseunterteil (18) ist mittels eines Halteelements oder Sicherheitsverschlusses (19) lösbar befestigt, insbesondere auf das Gehäuseinnenteil (17) aufgesteckt. Insbesondere ist das Halteelement oder der Sicherheitsverschluss (19) derart ausgebildet, dass ein versehentliches Öffnen des Zerstäubers (1) bzw. Abziehen des Gehäuseunterteils (18) ausgeschlossen ist. Insbesondere muss zum Lösen des Gehäuseunterteils (18) der Sicherheitsverschluss (19) gegen Federkraft eingedrückt werden. Der Sicherheitsverschluss (19) ist vorzugsweise eine zum Gehäuseinnenteil (17) gehörende und/oder an dieses angespritzte Form, die beispielsweise wie ein Federhaken gestaltet ist.
Alternativ zum Darstellungsbeispiel kann der Zerstäuber (1) auch so gestaltet sein, dass der Behälter (3) im Zerstäuber vorinstalliert ist. Bei dieser - hier nicht anhand der Figuren dargestellten - Variante mit voreingesetztem Behälter (3) wird dieser bereits ab Werk in den Zerstäuber (1) oder in ein zusätzliches Halte- oder Sicherungselement im Gehäuseunterteil (18) eingesetzt, das bei Auslieferung nur teilweise auf das Gehäuseinnenteil (17) aufgeschoben ist. Beim weiteren Aufschieben auf das Gehäuseinnenteil (17) gleitet das Gehäuseunterteils (18) beispielsweise über eine nur in eine Bewegungsrichtung gleitend gestaltete Ratschenbahn oder hakt nach vollständigem Aufschieben in eine variable gestaltbare Verrastung unlösbar ein. Gleichzeitig wird beim Aufschieben des Gehäuseunterteils der Behälter (3) in seine Halterung (6) geschoben und an den Hohlkolben (9) angeschlossen. Weitere Details zur Gestaltung solcher Systeme mit voreingesetztem Behälter (3) können der WO2006/125577A2 entnommen werden.

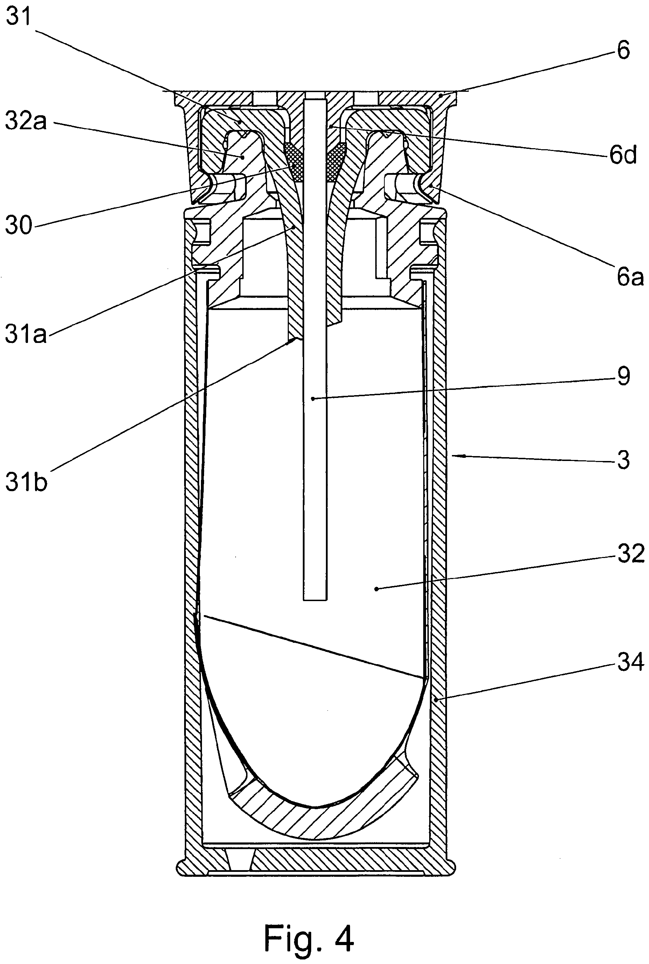

Vorzugsweise sind die hier betrachteten medizinischen Handgeräte auf die Ausbringung mehrerer Dosiseinheiten der flüssigen, medizinischen Formulierung ausgelegt. So zeigt der Zerstäuber (1) in Figur 1 ein Zählwerk (41) - in der konkreten Ausgestaltung ein durch die Gehäusedrehung angetriebenes Spindel-Reiter-Zählwerk, anhand dessen der (nicht dargestellte) Benutzer die Menge der entnommenen oder der im Gerät verbleibenden Dosiseinheiten ablesen kann. Der in das Gerät eingesetzte Behälter enthält eine Menge an Flüssigkeit (2), die für die Entnahme mehrerer - z.B. 30 bis 180 - Dosiseinheiten ausreicht. Aufgrund dieser mehrfachen Ausbringung muss der in den Zerstäuber (1) eingesetzte Behälter (3) so beschaffen sein, dass der Innendruck auch bei Flüssigkeitsentnahme im wesentlichen unverändert bleibt, so dass beim Ansaugen immer die gleiche Menge Flüssigkeit (2) entnommen wird. Hierzu kann prinzipiell sowohl ein Behälter (3) mit starrer Behälterwand, dessen Innendruck über eine Belüftung konstant gehalten wird und wie er beispielsweise in der WO2006/136426A1 beschrieben wird, als auch ein Behälter (3) mit einer flexiblen inneren Wand verwendet werden, die sich bei Flüssigkeitsentnahme zumindest teilweise derart ins Behälterinnere verschiebt und so durch Verkleinerung des Innenvolumens den Innendruck am Speicherort der Flüssigkeit konstant hält. Bevorzugt werden hierbei Behälter (3), in denen die flexible Wand durch einen Beutel (32) oder auch Innenbeutel oder Folienbeutel gebildet wird, der im Wesentlichen verformbar, zusammendrückbar und/oder zusammenziehbar ausgebildet ist. Solche Behälter werden in verschiedenen Ausführungsformen in den Schriften WO00/49988A2, WO01/076849A1, WO99/43571A1, WO2009/115200A1 und WO2009/103510A1 beschrieben.
Für die hier gewählten Darstellungsbeispiele, entsprechend denen auch in den Figuren 4 bis 9 die Behälteranbindung im Detail zu sehen sind, wird ein Behälter bevorzugt, in dem sich die Flüssigkeit (2) in einem flexiblem, verformbaren und/oder kollabierbaren Beutel (32) oder Schlauch befindet. Auf diese Weise kann der Innendruck am Speicherort der Flüssigkeit bei Flüssigkeitsentnahme konstant gehalten werden, ohne dass ein regelmäßiger Gasaustausch mit der Umgebung des Zerstäubers (1) stattfindet. Ein solcher Gasaustausch hätte insbesondere bei der Verwendung von flüssigen medizinischen Formulierungen mit leicht flüchtigen Lösemitteln wie z.B. Ethanol den Nachteil, dass bei jedem Belüftungsvorgang ein Lösemittelanteil über die Gasphase entweichen kann.

Entweicht Lösemittel über die Gasphase, bleibt weniger Lösemittel für die medizinische Formulierung im Behälter (3) zurück, und der Wirkstoff wird in der Flüssigkeit (2) aufkonzentriert. Durch diese Aufkonzentration würde beim Entnehmen einer abgemessenen Menge Flüssigkeit (2) eine relativ erhöhte Dosis des Wirkstoffes entnommen. Somit ist dieser Verlust von Lösemittel durch die Gasphase zu begrenzen oder nach Möglichkeit vermeiden. Dies ist eine der Forderungen an die Gestaltung des Behälters (3), die Wahl der verwendeten Materialien und die Gestaltung der Abdichtungen beim Einsetzen des Behälters (3) in das jeweilige Gerät bzw. in den Zerstäuber (1). Vorzugsweise wird für den die Flüssigkeit (2) aufnehmenden Beutel (32) als flexibles Wandmaterial eine mehrschichtig aufgebaute Folie oder dergleichen verwendet. Die Folie umfasst eine mit der medizinischen Flüssigkeit kompatible Kunststoffschicht und eine Metallschicht wie z.B. eine Aluminiumschicht oder ähnliches. Dadurch wird die Diffusion oder Permeation von Gas durch die Beutelwand hindurch minimiert.
Der für die Darstellungsbeispiele gewählte Behälter (3) umfasst einen inneren Beutel (32), einen Flansch (32a), eine Behälterkappe (31) und eine starre Hülse (34). Der flexible, mehrschichtige, unten geschlossene Beutel (32) ist im oberen Bereich direkt mit einem Halt gebenden Flansch (32a) vorzugsweise aus Kunststoff verbunden. Die starre Hülse (34) umgibt den Beutel (32) und schützt ihn vor mechanischen Beschädigungen von außen. Die Behälterkappe (31) ist vorzugsweise aus Kunststoff, besonders bevorzugt aus HD-PE, und insbesondere aus einem ähnlichen oder dem gleichen Material wie der Flansch (32a). Die Behälterkappe (31) wird nach Befüllen des Beutels (32) mit Flüssigkeit (2) vorzugsweise über einen Warmverformungsprozess oder einen Schweißprozess (z.B. Ultraschall- oder Laserschweißen) dicht mit dem Flansch (32a) verbunden.

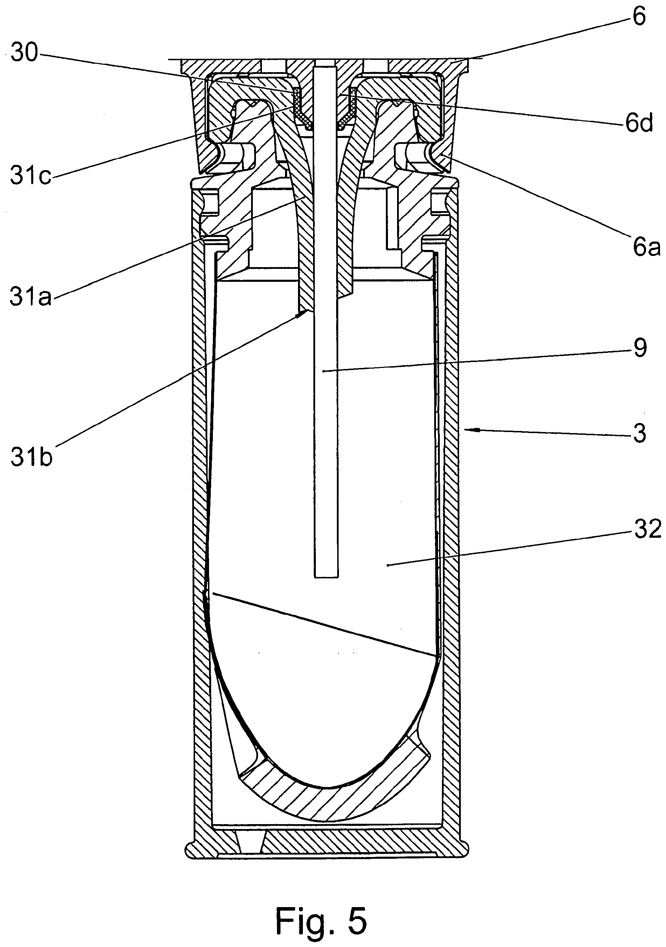

Die Behälterkappe (31) weist als Einführstelle einen in den Innenraum des Beutels (32) hineinragenden Einführtrichter (31a) auf, der bei Anschluss des Behälters an den Zerstäuber (1) eine zentrierte Führung für den Hohlkolben (9) bildet und somit ein bezogen auf die Verbindungsstelle unkontrolliertes Anstechen des Behälters (3) durch den Hohlkolben (9) verhindert. Vor der Anbindung an den Zerstäuber (1) ist der Behälter bzw. das dem Innenraum des Behälters (3) zugewandte Ende des Einführtrichters (31a) mit einer Membran (31b) verschlossen, die beim Einführen des Hohlkolbens (9) durchstochen oder aufgeklappt wird. Auf diese Weise schützt die Membran (31b) den nichtangestochenen Behälter vor Flüssigkeitsaustritt. Zusätzlich besteht die hier nicht in den Figuren dargestellte Möglichkeit, den Behälter während der Lagerung mit einem Kopfsiegel zu versehen, das beispielsweise aus einer Metallfolie vorzugsweise aus Aluminium bestehen kann und das obere offene Ende des Einführtrichters (31a) verschließt. Ein solches Siegel kann als Originalitätszeichen dienen und den Einführtrichter (31a) während des Transportes von einzelnen Kartuschen vor Verunreinigungen schützen. Gase, welche die Membran (31b) gegebenenfalls durchdringen können, werden von einem metallischen Kopfsiegel zurückgehalten. Vor Einbau des Behälters (3) in das Gerät kann das Kopfsiegel entfernt werden, z.B. durch Abziehen mittels einer überstehenden Lasche.
Nach kompletter Einführung des Behälters (3) in die Halterung (6) des Zerstäubers (1) liegt eine Presspassung zwischen dem eingeführten Rohr oder Hohlkolben (9) und der Wand der Einführtrichters (31a) vor. Diese Presspassung in einem Abschnitt des Einführtrichters (31a) bildet eine zur Behälterkappe (31) gehörende Dichtung, die hier auch als erste Dichtung bezeichnet wird. Die radial wirkende Presspassung dichtet die Kontaktstelle zwischen Hohlkolben (9) und dem Inneren des Behälters (3) gegen Flüssigkeitsverlust außen am Hohlkolben (9) vorbei über eine Länge von 1 bis 10 Millimetern, vorzugsweise 2 bis 7 Millimeter, besonders bevorzugt 5 mm ab. Im Darstellungsbeispiel ist der Hohlkolben (9) aus Metall, vorzugsweise aus Edelstahl. Die Behälterkappe (31) ist aus einem gegenüber dem Hohlkolben (9) weicheren KunststoffMaterial, vorzugsweise PE oder HD-PE. Das Material der Behälterkappe (31) darf jedoch nicht beliebig weich sein, da für die Funktionssicherheit des Systems Formstabilität wichtig ist. Aus diesem Grund kann die Presspassung zwischen Hohlkolben (9) und Einführtrichter (31a) zwar gegen Flüssigkeitsdurchlass dicht ausgelegt werden, nicht jedoch zwangsläufig auch gegen Gasdurchlässigkeit. Je nach Fertigungsverfahren des Hohlkolbens (9) können sich beispielsweise bis hin zu wenigen Mikrometer tiefen Riefen oder Unebenheiten auf seiner Oberfläche befinden, welche die Gaspermeation durch die Presspassung hindurch begünstigen. Aus diesem Grund ist hier eine zweite Dichtung (30) mit gegenüber der Presspassung veränderten Dichteigenschaften eingebaut, um entweder durch die Presspassung entweichendes Gas abzufangen oder im Vorfeld bereits Luft am Eindringen in das System von außen am Hohlkolben (9) vorbei zu hindern. Die Figur 4 zeigt eine erfindungsgemäße Ausgestaltung der zweiten Dichtung, die Figuren 5 bis 9 zeigen unterschiedliche Alternativen einer zweiten Dichtung (30). Halterung (6) und Hohlkolben (9) sind hierbei der Einfachheit halber nur in ihrem unteren Teil abgebildet (eine komplette Darstellung dieser Bauteile findet sich in den Figuren 1 bis 3).
Figur 4 zeigt ein Beispiel für die zweite Dichtung (30), die als zusätzliches dichtendes Bauteil außen an einem Rohr wie dem Hohlkolben (9) oder an der die Behälteraufnahme bildenden Halterung (6) am Gerät angebracht ist. Dargestellt ist hier eine radial symmetrische Dichtung (30), die im oberen Bereich des Einführtrichters (31a), d.h. in seinem aufgeweiteten Bereich oberhalb der Presspassung zum Hohlkolben (9), Behälterkappe (31) und Hohlkolben (9) gegeneinander abdichtet. Diese zweite - im Vergleich zur ersten Dichtung geräteseitige - Dichtung (30) umschließt den Hohlkolben (9) radial im Bereich der Halterung (6) - entweder direkt oder durch die Innenführung (6d) beabstandet. Die Dichtung (30) liegt hier an ihrer Oberseite an der Halterung (6), insbesondere an der Innenführung (6d), an, die ein Stück am Hohlkolben (9) entlang nach unten in Richtung Behälter (3) gezogen ist. Die als Bauteil gestaltete Dichtung (30) hat innen eine kreiszylindrische Aussparung für die Durchführung des Hohlkolbens (9), im oberen Bereich eine an die Form der Innenführung (6d) der Halterung (6) angepasste, in diesem Beispiel trichterförmige Gestalt und außen eine sich in Richtung Behälter (3) verjüngende konusförmige Gestalt. Diese konusförmige Gestalt bildet ein Gegenstück zur Innenkontur des Einführtrichters (31a) der Behälterkappe (31). Die Dichtung (30) taucht von der Halterung (6) mit dem Hohlkolben (9) geführt in die konusförmige Öffnung ein, die der Behälter (3) bzw. die Behälterkappe (31) für die Einführung des Hohlkolbens (9) aufweist. Im Einbauzustand dichtet die zweite Dichtung (30) im konusförmigen Wandbereich des Einführtrichters (31a) der Behälterkappe (31).
Vorzugsweise ist die Dichtung (30) den Hohlkolben (9) umgebend auf der Halterung (6) vormontiert, wobei sie durch die Innenführung (6d) abgestützt wird. Wird nun der Behälter (3) in den Zerstäuber (1) eingesetzt und dabei axial auf den Hohlkoben (9) aufgeschoben, so wird die Dichtung (30) zwischen Innenführung (6d) und der inneren Wand des Einführtrichters (31a) an der Behälterkappe (31) axial verpresst. Betrachtet man die Anbindung des Behälters (3) an das Gerät insgesamt, so wird eine Dichtungswirkung sowohl durch axiale Verpressung insbesondere parallel zum rohrförmigen Bauteil oder Hohlkolben (9) als auch durch radiale Verpressung insbesondere senkrecht zum Hohlkolben (9) erzielt. Durch die Kombination einer radial wirkenden Dichtung in Form der Presspassung zwischen Hohlkolben (9) und Behälterkappe (31) und der im wesentlichen axial wirkenden zusätzlichen Dichtung (30) zwischen Behälterkappe (31) und Behälteraufnahme wird eine doppelt wirkende Abdichtung des Systems erzielt. Die Dichtung (30) besteht vorzugsweise aus einem Elastomer wie Silizium- und/oder Kohlenstoff basierten elastomeren Polymeren. Geeignete Materialien umfassen natürliche und synthetische Elastomere, beispielsweise Nitril-Kautschuk, Butadien-Kautschuk, Styrol-Butadien-Kautschuk, Isopren-Kautschuk, Styrol-Isopren-Kopolymere, ButylKautschuk wie Isobuten-Isopren-Kautschuk, Polyurethan, Fluor-Kautschuk, Siloxane wie insbesondere Silikone und Diene wie insbesondere EPDM (Ethylen-Propylen-DienKautschuk) oder einem anderen für die Verwendung im medizinischen Bereich geeigneten Elastomer. Je nach Forderung, z.B. bei geforderten speziellen Materialresistenzen oder montagebedingten Gleiteigenschaften, kann die Dichtung zusätzlich beschichtet sein. So sind z.B. mit PTFE (Polytetrafluorethylen) beschichtete dichtende Bauteile aus Fluor-Kautschuk vor dem Hintergrund einer besseren Vereinzelbarkeit auf Montagemaschinen für die Massenfertigung vorteilhaft.
Die Verwendung eines weichen Elastomers, insbesondere eines mit einer Shore-Härte im Bereich von 40 bis 70 Shore, für die Dichtung (30) hat den Vorteil, dass sowohl gegenüber dem Einführtrichter (31a) als auch gegenüber dem Hohlkolben (9) eine Hart-Weich-Dichtung ausgebildet wird. Unebenheiten in den vergleichsweise harten Oberflächen von Einführtrichter (31a) und Hohlkolben (9) können somit von der Dichtung (30) ausgeglichen werden, so dass der Übergangsbereich zwischen den Bauteilen auch für flüchtige Substanzen dicht ist. Die Dichtung (30) kann somit auch als Gasphasendichtung bezeichnet werden, da hier die Permeation von Gasen am Hohlkolben (9) entlang unterbunden wird.
Die im speziellen Ausführungsbeispiel aus Figur 4 dargestellte Dichtung (30) hat zusätzlich den Vorteil, dass sie weit in den Einführtrichter (31a) hinein ragt und somit das Totvolumen, d.h. der ungenutzte freie Raum zwischen zweiter Dichtung (30), Hohlkolben (9) und erster Dichtung (Presspassung zwischen Hohlkolben (9) und Behälterkappe (31)) vergleichsweise klein gehalten wird. Dieses Totvolumen ist so lange konstant, wie der Behälter (3) im Gerät eingebaut ist. Die an der Dichtung beteiligten Bauteile werden beim Betrieb des Geräts oder Zerstäubers (1) nicht gegeneinander bewegt. Durch die Minimierung des Totvolumens stellt sich darin bei der Permeation von Gas aus dem Beutel (32) durch die Presspassung hindurch schnell ein Gleichgewicht ein, so dass die Gesamtmenge der durch Ausgasung verloren gehenden Substanzen sehr gering gehalten werden kann. Die Substanz mit dem höchsten Dampfdruck ist im Anwendungsbeispiel meist das Lösemittel der medizinischen Formulierung im Beutel (32). Durch die hier erzielte Minimierung der Lösemittel-Ausgasung wird eine Aufkonzentration des Wirkstoffes in der Lösung verhindert. Versuche zeigten eine gute Langzeitstabilität der Wirkung der zweiten Dichtung (30). Die gravimetrisch bestimmten Verdunstungswerte von in Geräte eingebauten Behältern (3) waren unter Verwendung der Dichtung (30) je nach ihrem Material ähnlich gering wie die von nicht angestochenen Behältern (3) mit intakter Membran (31b). Bei einem Gesamtvolumen von etwa 4 Millilitern des Beutels (32) lagen die Verdunstungswerte für Ethanol bei allen getesteten Dichtungsmaterialien und bei allen einzelnen Proben deutlich unter dem für dieses Experiment festgesetzten Grenzwert: Das durch die beiden Dichtungen zwischen Behälterkappe und Gerät gebildete Dichtsystem wies eine Ethanol-Permeabilität von weniger als 0,3 Milligramm pro Tag auf. Die erzielten Ergebnisse lagen im Mittel zwischen 0,005 und 0,04 Milligramm pro Tag. Selbst bei der Verwendung eines Elastomers wie Silikon, das an sich für seine Teildurchlässigkeit von Ethanol bekannt ist, lag der höchste gemessene Einzelwert unter 0,15 Milligramm pro Tag.
Figur 5 zeigt im Einbauzustand eine ebenfalls als Bauteil gestaltete zweite Dichtung (30), die eine erste Alternative zur erfindungsgemäßen Abdichtung bildet. Das Material der Dichtung (30) entspricht dem wie im Ausführungsbeispiel aus Figur 4. Ebenso wie dort sollte für den speziellen Anwendungsfall ein Material gewählt werden, das insbesondere diffusionsdicht gegenüber den in der medizinischen Formulierung enthaltenen Substanzen ist, also beispielsweise keine Gaspermeation des Lösemittels zulässt. Das in Figur 5 dargestellte, die Dichtung (30) bildende Bauteil ist ebenfalls radial symmetrisch und hat die Form eines unten geöffneten Bechers oder einer Kappe. Die Öffnung des Bechers ist dabei die Durchführung des Hohlkolbens (9). Die innere Form des dichtenden Bauteils bzw. die Innenkontur des Bechers ist das Gegenstück zur Form der Innenführung (6d) der Halterung (6) im Bereich des Hohlkolbens (9). Im speziellen Darstellungsbeispiel hat das dichtende Bauteil einen oberen kreiszylindrischen Bereich, der in einen innen sich nach unten konisch verjüngenden Bereich übergeht. Der Becherboden ist trichterförmig. Die Abdichtung erfolgt über eine rein radial wirkende Verpressung der Dichtung (30) zwischen der Innenführung (6a) der Halterung (6) und einem kreiszylindrischen Öffnungsbereich (31c) der Behälterkappe (31). Dieser kreiszylindrische Öffnungsbereich (31c) schließt sich an den Einführtrichter (31a) nach oben hin an der Behälterkappe (31) an. In diesem Ausführungsbeispiel werden also beide Dichtungen durch radiale Verpressung zwischen Behälter (3) bzw. Behälterkappe (31) und Hohlkolben (9) oder Halterung (6) gebildet. Die radiale Wirkung der Dichtung (30) hat den Vorteil, dass keine zusätzlichen axialen Kräfte beim Einsetzen des Behälters (3) in das Gerät aufgebracht werden müssen. Das die zweite Dichtung (30) bildende Bauteil ist hier derart geformt, dass es einen Abstützbereich aufweist, der in axialer Richtung an einem anderen Bauteil des Geräts insbesondere an der Halterung (6) anliegt. Die kappenartige oder becherartige Form des dichtenden Bauteils ist an die untere Kante der Innenführung (6d) der Halterung (6) angepasst und der innere Becherboden, der den Abstützbereich des dichtenden Bauteils darstellt, liegt unten an der Innenführung (6d) an. Der Abstützbereich des die Dichtung (30) bildenden Bauteils liegt somit auf dem in den Behälter (3) hineinragenden Bereich der Halterung (6) auf. Selbst bei der Verwendung von sehr weichen Dichtungsmaterialien wird das dichtende Bauteil durch diese Abstützung in Position gehalten und schiebt sich beim Andocken, d.h. beim Einsetzen des Behälters (3) in die Halterung (6), nicht durch die axial wirkenden Kräfte in den oberen axialen Zwischenraum zwischen Behälterkappe (31) und Halterung (6). Zur Reduzierung des Totvolumens zwischen zweiter Dichtung (30), Behälterkappe (31), Hohlkolben (9) und Presspassung (erster Dichtung) kann der nicht-dichtende, abgestützte untere Bereich des dichtenden Bauteils verbreitert werden. Vorzugsweise hat dieser untere Bereich des die Dichtung (30) bildenden Bauteils keinen direkten Wandkontakt zum Einführtrichter (31a) der Behälterkappe (31), so dass beim Andocken des Behälters (3) keine zusätzlichen axialen Kräfte wirken.

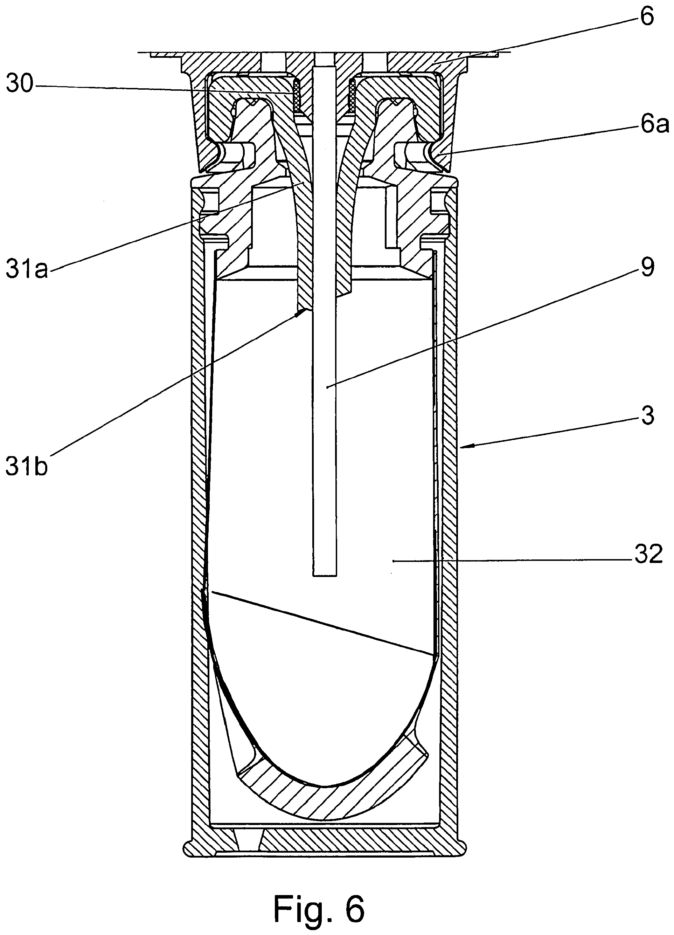

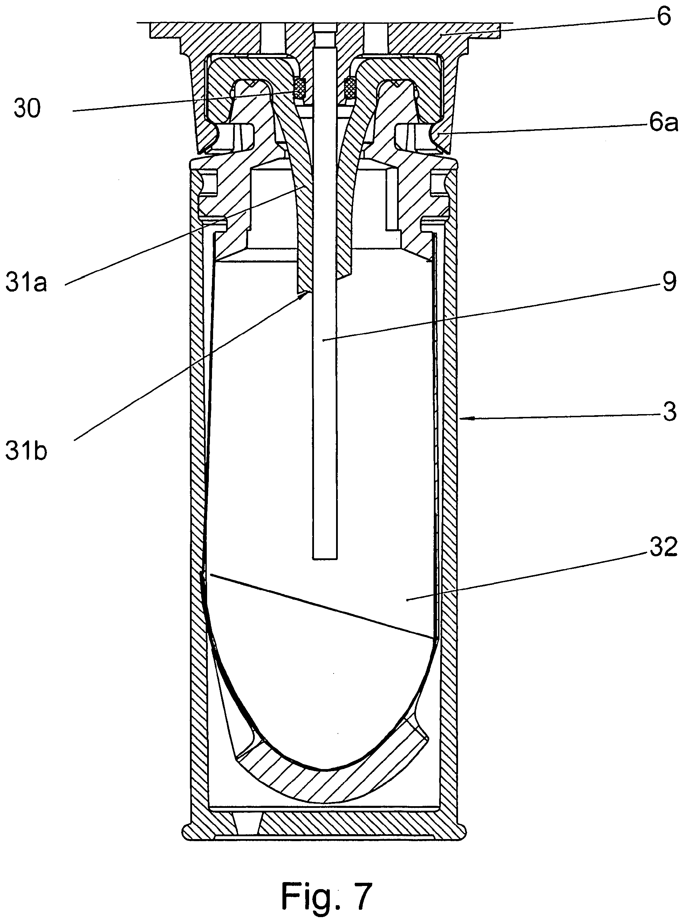

Figur 6 und 7 zeigen zwei weitere Dichtung (30) im Einbauzustand, die Alternativen zur erfindungsgemäßen Abdichtung bilden. Analog wie im Beispiel von Figur 5 wirkt hier die Abdichtung radial zwischen der Innenführung (6d) der Halterung (6) und der Innenwand der Behälterkappe (31), bzw. konkret im zylindrischen Öffnungsbereich (31c) oberhalb des Einführtrichters (31a). Durch die radiale Wirkung der Dichtung (30) müssen keine nennenswerten zusätzlichen axialen Kräfte beim Einsetzen des Behälters (3) in das Gerät aufgebracht werden. Die Abdichtung ist zu beiden Seiten eine Hart-Weich-Dichtung, bei der das oberflächenweiche Material der Dichtung (30) die Oberflächenunebenheiten der Behälterkappe bzw. der Innenführung (6d) der Halterung (6) im Dichtungsbereich ausgleicht. Die Dichtung (30) kann die Form einer flachen Ringdichtung haben, die über die Innenführung (6d) der Halterung (6) übergestülpt ist (Figur 6), oder sie kann die Form einer in die Innenführung (6d) eingelassenen flachen oder O-Ring-förmigen Dichtung (30) haben (Figur 7), die durch eine radiale Vertiefung an der Innenführung (6d) in Position gehalten wird. Weiter kann die Innenführung selbst nach unten konisch zulaufen, und die Dichtung (30) - an die Form der Innenführung angepasst - kann ein nach unten und nach innen verbreiterter Ring sein, der sich nach innen an der Innenführung (6d) abstützt (nicht in Figuren dargestellte Variante).
Ein bevorzugtes Montageverfahren sowohl für die Dichtung (30) gemäß der Ausführungsform aus Figur 4 als auch für Dichtungen gemäß der Beispiele aus Figur 5 bis 7 gestaltet sich wie folgt:
Zunächst wird der Hohlkolben (9) fest mit der Halterung (6) verbunden, vorzugsweise indem das Kunststoffmaterial der Halterung (6) direkt in einem Einlege-Spritzgussverfahren an den Hohlkolben (9) angespritzt wird. Dann wird die Halterung (6) mit dem Hohlkolben (9) im behälterseitig (d.h. hier unten) offenen, aber ansonsten fertig montierten Zerstäuber (1) angebracht. Vor oder bevorzugt nach der Montage der Halterung (6) im Zerstäuber (1) wird das die Dichtung (30) bildende, radialsymmetrische Bauteil von unten zentriert am Hohlkolben (9) entlang in seine Position an der Halterung (6) bzw. an der Innenführung (6d) geschoben. Dieser Vorgang erfolgt bevorzugt berührungsfrei, um keine Beschädigungen am Hohlkolben wie beispielsweise Riefen oder andere Unebenheiten zu erzeugen, welche die Wirksamkeit der ersten Dichtung, im Darstellungsbeispiel die Dichtung durch die Presspassung zwischen Hohlkolben (9) und Behälterkappe (31), schwächen können.
Für eine berührungsfreie Montage wird für die Dichtung (30) ein Material mit Reißdehnung von mindestens 200%, bevorzugt mit einer Reißdehnung zwischen 300% und 500% gewählt. In diesem Zusammenhang bedeutet dies, dass das radialsymmetrische Bauteil hinsichtlich seines Durchmessers um mindestens das Doppelte, bevorzugt das dreibis fünffache ausgeweitet werden kann, ohne dass sich Risse bilden. Das Material muss darüber hinaus so gewählt sein, dass das Bauteil bei dieser Belastung eine rein elastische Verformung durchläuft und anschließend wieder in seine Ursprungsform zurückkehrt. Die Montage des die Dichtung (30) bildenden Bauteils erfolgt vorzugsweise mittels eines Vorrichtung, bei der mindestens drei Greifarme in die kreisförmige Durchführung des Bauteils hineinragen und dieses von innen nach außen aufspreizen. Das aufgespreizte Bauteil wird über den Hohlkolben (9) in seine Position an der Halterung (6) geschoben. Eine innen zwischen den Greifern liegende Kunststoffhülse kann hierbei zusätzlich dem Schutz des Hohlkolbens dienen. Sobald das die Dichtung (30) bildende Bauteil seine axiale Position an Hohlkolben (9) bzw. Halterung (6) erreicht hat, wird eine äußere Hülse vorgeschoben, die das Bauteil beim Zurückziehen der Greifarme von diesen herunter schiebt. Je nach Form der des dichtenden Bauteils bzw. je nach Vorhandensein eines Abstützbereichs, kann eine weitere Feinjustierung der Position des die Dichtung (30) bildenden Bauteils auch beim Andocken des Behälters (3) an den Zerstäuber (1) erfolgen, wenn Behälterkappe (31), Dichtung (30) und Halterung (6) ggf. axial weiter zusammen geschoben werden. Nach Montage des die Dichtung (30) bildenden Bauteils im vormontierten Zerstäuber (1), kann dieser vor Auslieferung wahlweise mit einem Gehäuseunterteil (18) ohne Behälter verschlossen oder vorzugsweise mit einem nur teilweise angedockten Behälters (3) und Gehäuseunterteil (18) zu einem vorinstallierten System vervollständigt werden.

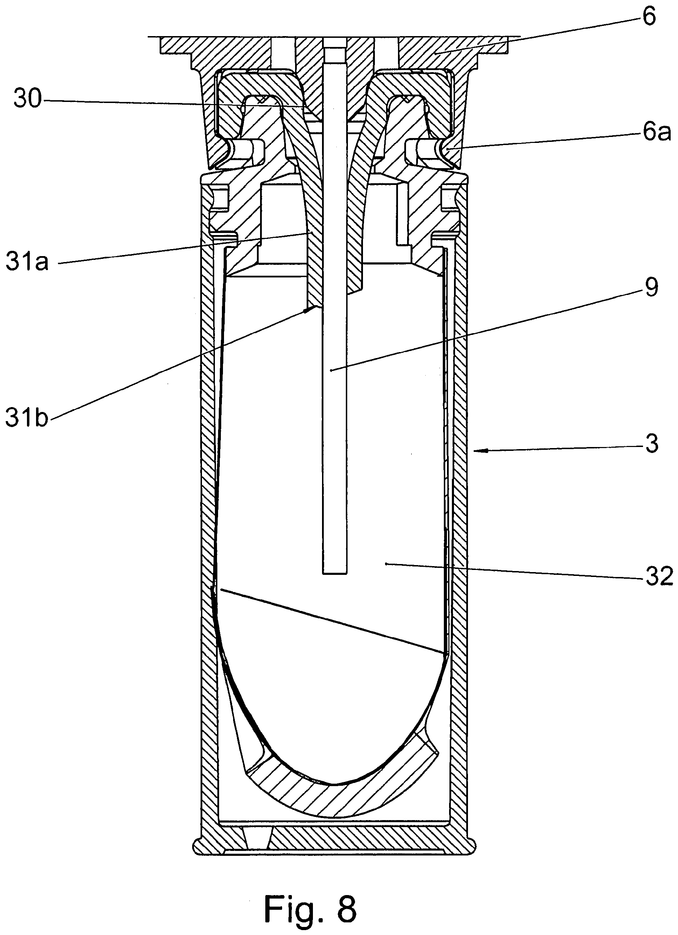

Figur 8 zeigt als vierte Alternative zur erfindungsgemäßen Abdichtung eine Dichtung (30), bei der die Dichtwirkung durch die Passform von Halterung (6) und Behälterkappe (31) erzielt wird. Hierbei ist die Halterung (6) bzw. ihre Innenführung (6d) derart geformt, dass sie selbst durch eine direkte Auflage an der Behälterkappe (31) die Dichtung (30) bildet. Dies ist vor allem im Hinblick auf Produktionskosten und Montageverfahren vorteilhaft, da die Dichtung (30) ohne den Einbau eines zusätzlichen Bauteils gebildet werden kann. Die Halterung (6) umschließt den Hohlkolben (9) entlang eines mittleren Abschnitts und ist unlösbar insbesondere durch Anspritzen mit diesem verbunden ist. Die am Hohlkolben anliegende Innenführung (6d) ist in allen Ausführungsbeispielen im Vergleich zum gesamten Durchmesser der Halterung (6) deutlich schmaler und ragt nach Anschluss des Behälters (3) an die Halterung (6) in diesen hinein. Die Unterkante der Innenführung (6d) liegt dann niedriger als die Oberkante des Behälters (3). Die Innenführung taucht stückweit in den Einführtrichter (31a) der Behälterkappe (31) ein.
Je nach Wahl der Materialien der in Figur 8 gezeigten Alternative handelt es sich bei dieser zweiten Dichtung (30) um eine Hart-Hart-Dichtung, da aufgrund der Forderung an Rückhalteeigenschaften insbesondere der Halterung (6) sowie der Behälterkappe (31) beide Bauteile eine gewissen Steifigkeit haben müssen. Für die Behälterkappe (31) kann in diesem Zusammenhang zwar ein oberflächen-weicheres Material als für die Halterung (6) gewählt werden, eine gewisse Steifigkeit der Behälterkappe (31) muss jedoch gewährleistet sein. Im Darstellungsbeispiel ist die Innenführung (6d) so gestaltet, dass sie im oberen Öffnungsbereich an der Behälterkappe (31) anliegt. Hierbei kann sie analog zum Beispiel von Figur 6 und 7 die Abdichtung im zylindrischen Öffnungsbereich (31c) bilden oder, wie in Figur 8 dargestellt, die Abdichtung im Eingangsbereich des zylindrischen Öffnungsbereichs (31c) bilden, d.h. in jenem fertigungstechnisch abgerundeten Oberflächenbereich, an dem die Kopfseite der Behälterkappe (31) in den kreiszylindrischen Öffnungsbereich (31c) übergeht. In einer weiteren, nicht bildlich dargestellten Beispiel kann die Innenführung (6d) auch direkt im oberen Bereich des Einführtrichters (31a) anliegen. In diesem Fall bildet sie vorzugsweise eine umlaufende, spitze Kante, die sich zur besseren Abdichtung in die Oberfläche des Einführtrichters (31a) d.h. in die nach innen zulaufende Schräge eindrückt. Diese Kante ist vorzugsweise nicht nur in radialer Richtung, sondern auch in axialer Richtung spitz zulaufend, d.h. die äußere untere Kante der Innenführung (6d) ragt weiter in Richtung des Behälter (3) als der durch Umspritzung gebildete Materialkontakt zwischen Innenführung (6d) und Hohlkolben (9). Im Bereich des Hohlkolbens (9) weist die Innenführung (6d) also in diesem nicht dargestellten Beispiel einen Hinterschnitt auf. Diese durch die Innenführung (6d) gebildeten Dichtungen (30) wirken bevorzugt axial, so dass sie wie bei Darstellungsbeispiel aus Figur 4 eine geeignete Kombination zur radial wirkenden, abdichtenden Presspassung zwischen Hohlkolben (9) und Behälterkappe (31) bilden.

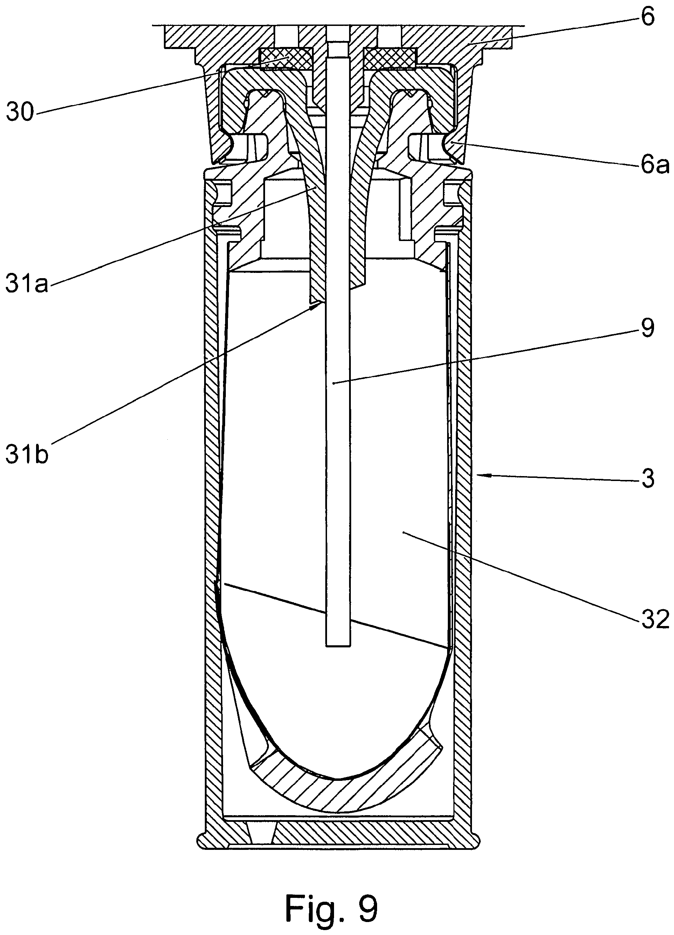

Figur 9 zeigt als fünfte Alternative zur erfindungsgemäßen Abdichtung eine zweiten Dichtung (30) im Einbauzustand, bei der die Dichtung (30) als eigenständiges Bauteil an der Kopfseite des Behälters in die Halterung (6) eingelassen oder eingelegt ist. Die Dichtung (30) hat die Form einer radial symmetrischen Flachdichtung, vorzugsweise einer flachen Ringdichtung, die gegebenenfalls mit inneren Einführschrägen versehen ist. Das Material der Dichtung (30) entspricht dem im Ausführungsbeispiel aus Figur 4. Die Dichtung (30) wirkt durch ihre Verpressung vorwiegend axial (parallel zum Rohr oder Hohlkolben (9)), bildet also eine geeignete Kombination zur radial wirkenden, abdichtenden Presspassung zwischen Hohlkolben (9) und Behälterkappe (31). Die Abdichtung wird bei der Verpressung zwischen Halterung (6) und Kopfseite der Behälterkappe (31) beim Andocken des Behälters (3) in der Halterung (6) gebildet. Innen liegt das die Dichtung (30) bildende Bauteil an der Innenführung (6d) an.
Alternativ zu den in den Figuren gezeigten Beispielen kann die zweite Dichtung auch durch eine Dichtungsschicht gebildet werden - einen an die Halterung (6) zusätzlich angespritzten Bereich, dessen Material sich von dem der Halterung (6) unterscheidet. Dieser zusätzliche Materialbereich kann aus einem elastomeren Material bestehen und ähnliche Bereiche an der Halterung (6) ausfüllen wie die eigenständigen elastomeren Bauteile in der Ausführungsform gemäß Figur 4 und den alternativen Beispielen in Figur 5 bis 7 und 9. Das Material ist weicher als das von Behälterkappe und Halterung. Diese Dichtungsschicht kann aus einem der oben genannten Elastomermaterialien insbesondere aber aus einem thermoplastischen Elastomer (TPE) wie beispielsweise auf Basis von Urethan oder Olefinen gebildet sein. Die Dichtstellen und die Wirkung der Dichtung entsprechen je nach gewähltem Anspritzbereich denjenigen der Ausführungsform in Figur 4 oder den Beispielen aus Figur 9 und Figur 5 bis 7. Ist also in den bisher dargestellten Beispielen von einer Dichtung (30) die Rede gewesen, die am Gerät "angebracht" ist, so schließt diese Ausdrucksweise neben den am Gerät montierten Einzelbauteilen auch solche Dichtungsschichten ein, die einstückig mit anderen Gerätebauteilen verbunden sind. Alternativ kann die Dichtungsschicht auch ein an den Behälter (3) oder an die Behälterkappe (31) zusätzlich angespritzter Bereich aus einem der angesprochenen elastomeren Materialien sein. In diesem Fall befindet sich die Dichtungsschicht entweder am Innenrand der Einführstelle bzw. an der Innenwand des Einführtrichters (31a) oder im oberen Bereich der Behälterkappe (31). Die Dichtungsschicht kann beispielsweise als eine oder mehrere angespritzte Lasche gestaltet sein, die vor Einsetzen des Behälters (3) in die Halterung (6) nach oben ragt und dann beim Einsetzen von Konturen der Halterung (6) nach innen in den Zwischenraum zwischen Behälterkappe (31) und Innenführung (6d) gedrückt wird. Eine solche an der Behälterkappe (31) angebrachte Dichtungschicht hat insbesondere bei wiederverwertbaren Geräten - z.B. bei einem Zerstäuber (1), der nach einander mit mehreren Behältern (3) betrieben wird - den Vorteil, dass jede Dichtung (30) nur einmal benutzt wird und somit keine Vorschädigung erfahren kann. Jeder Behälter (3) bringt ein neues, unbenutztes Dichtsystem aus erster und zweiter Dichtung mit ins Gerät ein.

In einem weiteren, nicht gezeigten Alternativsbeispiel, in dem die zweite Dichtung (30) ähnlich wie Darstellungsbeispiel von Figur 8 wirkt, weist die Halterung (6) einen zusätzlichen Materialbereich auf, der härter ist als das Material der Behälterkappe (31). Beispielsweise handelt es sich bei diesem zusätzlichen Materialbereich um ein Einlegeteil im Mehrkomponentenspritzguss. Dieses Einlegeteil, vorzugsweise aus einem harten Metall, könnte sich am unteren Ende der Innenführung (6d) axial besser in das Material der Behälterkappe (31) im Einführtrichter (31a) eindrücken oder - im Falle einer Einmal-Paarung von Gerät und Behälter - einschneiden als der jeweils für die Umspritzung des Hohlkolbens (9) für die Halterung (6) gewählte Kunststoff, der eine gewisse Elastizität hinsichtlich der Beschaffenheit der Schnapphaken (6a) aufweisen muss.

Der hier dargestellte treibgasfreie Zerstäuber dient der Ausgabe einer flüssigen medizinischen Formulierung als inhalierfähiges Aerosol und eignet sich für die Ausbringung sowohl wässriger als auch bevorzugt alkoholischer, insbesondere ethanolischer, medizinischer Formulierungen. Insbesondere wird hier eine zu verabreichende flüssige medizinische Formulierung eingesetzt, die eine Substanz mit hohem Dampfdruck oder eine alkoholische Verbindung enthält.

Bevorzugte Inhaltsstoffe der vorzugsweise flüssigen medizinischen Formulierung sind insbesondere in den Schriften WO09/047173A2 und WO09/115200A1 aufgeführt.

Insbesondere kann es sich bei dem in diesen Schriften beschriebenen Fluid um wässrige oder nicht wässrige Lösungen, Mischungen, Formulierungen mit und ohne Lösungsmittel, wie Ethanol oder dergleichen, handeln.

Der Vorschlag, die Verbindungsstelle eines Behälters mit einem der Ausbringung von Flüssigkeit dienenden Gerät mit einer doppelten Dichtung gegen Flüssigkeits- und Gasverlust auszustatten, ist auf viele Geräte, in denen Flüssigkeiten gefördert oder transportiert werden, übertragbar. Insbesondere ist die Erfindung auf alle Arten von Dosierziehern gerichtet, d.h. auf Geräte, aus denen mit jeder Entnahme eine vordefinierte Menge an Flüssigkeit aus einem Behälter gezogen wird. Des Weiteren arbeitet der vorschlagsgemäße Zerstäuber (1) mechanisch, während das hier vorgestellte Dichtsystem nicht auf die Anwendung in rein mechanischen Geräten zur Ausbringung einer Flüssigkeit begrenzt ist. Es kann beispielsweise ebenso in Systemen eingesetzt werden, in denen die Flüssigkeit durch elektrische, hydraulische oder andere Pumpen oder durch Treibmittel ausgebracht wird. Begriffe wie "Druckerzeuger" sind also allgemein zu verstehen. In diesem Sinne kann die vorliegende Erfindung auch Gebiets-übergreifend verwendet werden; selbst Anwendungen über den medizinischen Bereich hinaus sind möglich.

**Bezugszeichenliste**

| | | | |
|---|---|---|---|
| 1 | Zerstäuber | 23 | Zentralteil |
| 2 | Flüssigkeit | 24 | Dichtung |
| 3 | Behälter | 25 | Stützring |
| 5 | Druckerzeuger | 26 | Überwurfmutter |
| 6 | Halterung (für Behälter) | 27 | Vorfilter |
| 6a | Schnapphaken (an Halterung) | 28 | Feinfilter |
| 6b | Einführschräge (an Schnapphaken) | | |
| 6c | Wulst (an Schnapphaken) | 29 | Düsenbaugruppe |
| 6d | Innenführung (an Halterung) | | |
| 7 | Antriebsfeder | 30 | Dichtung |
| 8 | Sperring | 31 | Behälterkappe |
| 9 | Hohlkolben | 31a | Einführtrichter (inBehälterkappe) |
| 10 | Rückschlagventil | | |
| 11 | Druckkammer | 31b | Membran (in Behälterkappe) |
| 12 | Düse | 31c | zylindrischer Öffnungsbereich (inBehälterkappe) |
| 12a | Düsenkanäle | | |
| 12b | Feinstfilter | 32 | Beutel |
| 14 | Aerosol | 32a | Flansch (am Beutel) |
| 16 | Gehäuseoberteil | 34 | Hülse |
| 17 | Gehäuseinnenteil | 40 | Taste |
| 18 | Gehäuseunterteil | 41 | Zählwerk |
| 19 | Sicherheitsverschluss | | |

## Patentansprüche

1. Gerät zum Verabreichen einer flüssigen medizinischen Formulierung, die sich in einem in das Gerät eingesetzten Behälter (3) befindet, wobei der Behälter innerhalb des Geräts auf ein steifes Rohr aufgeschoben ist und dieses Rohr mit der Halterung (6) für den Behälter (3) im Gerät verbunden ist und wobei der Behälter (3) eine Einführstelle aufweist, die sich am Behälter (3) an einer zu diesem gehörenden Behälterkappe (31) befindet und an der eine erste Dichtung in Form einer Passdichtung zwischen Behälter (3) und einem Abschnitt des steifen Rohres vorliegt, wobei die erste Dichtung zwischen der Behälterkappe (31) und dem steifen Rohr gebildet wird und die Einführstelle am Behälter (3) als Einführtrichter (31a) ausgebildet ist, wobei dieser Einführtrichter (31a) einen konusförmigen Öffnungsbereich hat,
**dadurch gekennzeichnet, dass** zwischen Halterung (6) und Behälter (3) eine zweite Dichtung (30) vorliegt, die einen Raum zwischen erster Dichtung, Behälter (3) und Rohr gegen den Austritt von Flüssigkeit und Gasen und/oder gegen das Eindringen von Gasen abdichtet, wobei die zweite Dichtung (30) durch ein zusätzliches Bauteil oder durch eine Dichtungsschicht gebildet wird und sich dieses Bauteil oder diese Dichtungsschicht zwischen Halterung (6) und der Behälterkappe (31) befindet, und dass die zweite Dichtung (30) in dem konusförmigen Wandbereich des Einführtrichters (31a) dichtet.

2. Gerät nach Anspruch 1 **dadurch gekennzeichnet, dass** die erste Dichtung im wesentlichen dicht gegenüber den flüssigen Bestandteilen der medizinischen Formulierung im Behälter (3) ist und die zweite Dichtung (30) im wesentlichen dicht gegenüber Gasen ist.

3. Gerät nach Anspruch 1 oder 2 **dadurch gekennzeichnet, dass** die zweite Dichtung (30) durch ein elastomeres Bauteil gebildet wird, das zwischen der Halterung (6) und der Behälterkappe (31) angebracht ist und beim Andocken des Behälters (3) verpresst wird.

4. Gerät nach Anspruch 1 oder 2 **dadurch gekennzeichnet, dass** die zweite Dichtung (30) durch eine Dichtungsschicht gebildet wird, die sich an der dem Behälter (3) zugewandten Seite der Halterung (6) befindet und/oder die durch einen an die Halterung (6) oder an eine zur Halterung (6) gehörende Innenführung (6d) zusätzlich angespritzten Bereich gebildet wird, dessen Material weicher ist als das der Behälterkappe (31).

5. Gerät nach einem der Ansprüche 1 oder 2 **dadurch gekennzeichnet, dass** die zweite Dichtung durch eine Dichtungsschicht gebildet wird, die durch einen im oberen Bereich der Behälterkappe (31), in der sich die Einführstelle des Behälters (3) befindet, zusätzlich angespritzten Bereich gebildet wird, dessen Material weicher ist als das Material der Behälterkappe (31).

6. Gerät nach Anspruch 1 oder 2 **dadurch gekennzeichnet, dass** die zweite Dichtung (30) das Rohr direkt oder durch eine Innenführung (6d) an der Halterung (6) beabstandet radial umschließt.

7. Gerät nach Anspruch 1, 2 oder 6 **dadurch gekennzeichnet, dass** das die zweite Dichtung (30) bildende Bauteil oder die Dichtungsschicht vor Einsetzen des Behälters (3) in das Gerät oder in die Halterung (6) geräteseitig am Rohr oder an der die Behälteraufnahme bildenden Halterung (6) im Gerät angebracht ist.

8. Gerät nach einem der vorherigen Ansprüche **dadurch gekennzeichnet, dass** die zweite Dichtung (30) axial insbesondere parallel zum Rohr verpresst ist.

9. Gerät nach einem der Ansprüche 1 bis 7 **dadurch gekennzeichnet, dass** die zweite Dichtung (30) radial insbesondere senkrecht zum Rohr oder senkrecht zu einer Innenführung (6d) an der Halterung (6) verpresst ist.

10. Gerät nach einem der Ansprüche 3 oder 7 **dadurch gekennzeichnet, dass** das die zweite Dichtung (30) bildende Bauteil eine kappenartige oder becherartige oder hülsenartige oder konusartige Form mit Durchlassöffnung für das Rohr aufweist.

11. Gerät nach einem der Ansprüche 3 oder 7 **dadurch gekennzeichnet, dass** das die zweite Dichtung (30) bildende Bauteil eine O-Ring-Dichtung, eine Flachdichtung oder eine Ringdichtung ist.

12. Gerät nach einem der Ansprüche 3, 7, 10 oder 11 **dadurch gekennzeichnet, dass** das die zweite Dichtung (30) bildende Bauteil derart geformt ist, dass sie einen Abstützbereich aufweist, der in axialer Richtung an der Halterung (6), insbesondere an einer Innenführung (6d) der Halterung (6), anliegt.

13. Gerät nach einem Ansprüche 1 bis 12 **dadurch gekennzeichnet, dass** das Rohr der Kolben einer Hochdruckpumpe ist.

14. Gerät nach einem der vorherigen Ansprüche **dadurch gekennzeichnet, dass** die zu verabreichende flüssige medizinische Formulierung eine Substanz mit hohem Dampfdruck oder eine alkoholische Verbindung enthält.

15. Verwendung des Gerätes nach einem der vorherigen Ansprüche **dadurch gekennzeichnet, dass** die flüssige medizinische Formulierung durch eine Düse (12) am Gerät als Aerosol insbesondere als inhalierfähiges Aerosol verabreicht wird.

## Claims

1. Device for administering a liquid medicinal formulation which is located in a container (3) inserted in the device, wherein the container inside the device is pushed onto a rigid tube and this tube is connected to the holder (6) for the container (3) in the device, and wherein the container (3) has an insertion point, which is located on the container (3) on a container cap (31) belonging to the latter and at which a first seal in the form of a fitting seal is provided between the container (3) and a portion of the rigid tube, wherein the first seal is formed between the container cap (31) and the rigid tube and the insertion point on the container (3) is configured as an insertion funnel (31a), wherein this insertion funnel (31a) has a conical opening region,
**characterised**
**in that** between the holder (6) and the container (3) is a second seal (30) which seals off a space between the first seal, the container (3) and the tube to prevent the escape of liquids and gases and/or to prevent the ingress of gases, wherein the second seal (30) is formed by an additional component or by a sealing layer and this component or this sealing layer is located between the holder (6) and the container cap (31), and
**in that** the second seal (30) seals in the conical wall region of the insertion funnel (31a).

2. Device according to claim 1 **characterised in that** the first seal is substantially leaktight against the liquid components of the medicinal formulation in the container (3) and the second seal (30) is substantially leaktight against gases.

3. Device according to claim 1 or 2, **characterised in that** the second seal (30) is formed by an elastomeric component which is arranged between the holder (6) and the container cap (31) and is compressed as the container (3) is docked.

4. Device according to claim 1 or 2, **characterised in that** the second seal (30) is formed by a sealing layer which is located on the side of the holder (6) facing the container (3) and/or which is formed by a region additionally moulded onto the holder (6) or onto an inner guide (6d) belonging to the holder (6), the material of which region is softer than that of the container cap (31).

5. Device according to one of claims 1 or 2, **characterised in that** the second seal is formed by a sealing layer which is formed by a region additionally moulded on in the upper region of the container cap (31), in which the insertion point of the container cap (31) is located, wherein the material of the region additionally moulded on in the upper region of the container cap is softer than the material of the container cap (31).

6. Device according to claim 1 or 2, **characterised in that** the second seal (30) radially surrounds the tube directly or spaced apart from it by an inner guide (6d) on the holder (6).

7. Device according to claim 1, 2 or 6, **characterised in that** the component that forms the second seal (30) or the sealing layer before the insertion of the container (3) in the device or in the holder (6) is mounted at the device end on the tube or on the holder (6) that forms the container receptacle in the device.

8. Device according to one of the preceding claims, **characterised in that** the second seal (30) is axially compressed, particularly parallel to the tube.

9. Device according to one of claims 1 to 7, **characterised in that** the second seal (30) is radially compressed particularly perpendicularly to the tube or perpendicularly to an inner guide (6d) on the holder (6).

10. Device according to one of claims 3 or 7, **characterised in that** the component that forms the second seal (30) has a cap-shaped or cup-shaped or conical shape with a through-opening for the tube.

11. Device according to one of claims 3 or 7, **characterised in that** the component that forms the second seal (30) is an O-ring seal, a flat seal or a ring seal.

12. Device according to one of claims 3, 7, 10 or 11, **characterised in that** the component that forms the second seal (30) is shaped so that it comprises a support region which abuts in the axial direction on the holder (6), particularly on an inner guide (6d) of the holder (6).

13. Device according to one of claims 1 to 12, **characterised in that** the tube the piston is a high pressure pump.

14. Device according to one of the preceding claims, **characterised in that** the liquid medicinal formulation that is to be administered contains a substance with a high vapour pressure or an alcoholic compound.

15. Use of the device according to one of the preceding claims, **characterised in that** the liquid medicinal formulation is administered as an aerosol, particularly an inhalable aerosol, through a nozzle (12) at the device.

## Revendications

1. Appareil pour l'administration d'une formulation médicamenteuse liquide, qui se trouve dans un récipient (3) inséré dans l'appareil, dans lequel le récipient est poussé sur un tube rigide à l'intérieur de l'appareil et ce tube est relié à l'élément de retenue (6) pour le récipient (3) dans l'appareil et dans lequel le récipient (3) présente un point d'introduction, qui se trouve sur le récipient (3) sur un capuchon de récipient (31) faisant partie de celui-ci et au niveau duquel un premier joint sous forme d'un joint d'ajustage est présent entre le récipient (3) et une partie du tube rigide, dans lequel le premier joint est formé entre le capuchon de récipient (31) et le tube rigide et le point d'introduction sur le récipient (3) est réalisé en tant qu'entonnoir d'introduction (31a), dans lequel cet entonnoir d'introduction (31a) présente une zone d'ouverture conique,
**caractérisé en ce qu'**un deuxième joint (30), qui étanchéifie un espace entre le premier joint, le récipient (3) et le tube contre la sortie de liquide et de gaz et/ou contre la pénétration de gaz, est présent entre l'élément de retenue (6) et le récipient (3), dans lequel le deuxième joint (30) est formé par une pièce additionnelle ou par une couche d'étanchéité et cette pièce ou cette couche d'étanchéité se trouve entre l'élément de retenue (6) et le capuchon de récipient (31), et que le deuxième joint (30) assure une étanchéité dans la zone de paroi conique de l'entonnoir d'introduction (31a).

2. Appareil selon la revendication 1, **caractérisé en ce que** le premier joint est sensiblement étanche par rapport aux constituants liquides de la formulation médicamenteuse dans le récipient (3) et le deuxième joint (30) est sensiblement étanche vis-à-vis des gaz.

3. Appareil selon la revendication 1 ou 2, **caractérisé en ce que** le deuxième joint (30) est formé par une pièce élastomère, qui est montée entre l'élément de retenue (6) et le capuchon de récipient (31) et est pressée lors de la fixation du récipient (3) .

4. Appareil selon la revendication 1 ou 2, **caractérisé en ce que** le deuxième joint (30) est formé par une couche d'étanchéité, qui se trouve sur la face de l'élément de retenue (6) tournée vers le récipient (3) et/ou qui est formée par une zone, moulée par injection de manière additionnelle sur l'élément de retenue (6) ou sur un guidage intérieur (6d) faisant partie de l'élément de retenue (6), dont le matériau est plus souple que le capuchon de récipient (31).

5. Appareil selon l'une quelconque des revendications 1 ou 2, **caractérisé en ce que** le deuxième joint est formé par une couche d'étanchéité, qui est formée par une zone, moulée par injection de manière additionnelle dans la zone supérieure du capuchon de récipient (31), dans laquelle se trouve le point d'introduction du récipient (3), dont le matériau est plus souple que le matériau du capuchon de récipient (31).

6. Appareil selon la revendication 1 ou 2, **caractérisé en ce que** le deuxième joint (30) entoure radialement de manière espacée le tube de façon directe ou par un guidage intérieur (6d) sur l'élément de retenue (6).

7. Appareil selon la revendication 1, 2 ou 6, **caractérisé en ce que** la pièce formant le deuxième joint (30) ou la couche d'étanchéité, avant l'insertion du récipient (3) dans l'appareil ou dans l'élément de retenue (6), est montée côté appareil sur le tube ou sur l'élément de retenue (6) formant le logement de récipient.

8. Appareil selon l'une quelconque des revendications précédentes, **caractérisé en ce que** le deuxième joint (30) est pressé axialement, en particulier parallèlement, par rapport au tube.

9. Appareil selon l'une quelconque des revendications 1 à 7, **caractérisé en ce que** le deuxième joint (30) est pressé radialement, en particulier perpendiculairement, par rapport au tube ou perpendiculairement à un guidage intérieur (6d) sur l'élément de retenue (6).

10. Appareil selon l'une quelconque des revendications 3 ou 7, **caractérisé en ce que** la pièce formant le deuxième joint (30) présente une forme du type capuchon ou du type godet ou du type manchon ou du type cône avec ouverture de passage pour le tube.

11. Appareil selon l'une quelconque des revendications 3 ou 7, **caractérisé en ce que** la pièce formant le deuxième joint (30) est un joint torique, un joint plat ou un joint annulaire.

12. Appareil selon l'une quelconque des revendications 3, 7, 10 ou 11, **caractérisé en ce que** la pièce formant le deuxième joint (30) est formée de telle sorte qu'il présente une zone d'appui, qui s'applique dans la direction axiale sur l'élément de retenue (6), en particulier sur un guidage intérieur (6d) de l'élément de retenue (6).

13. Appareil selon l'une quelconque des revendications 1 à 12, **caractérisé en ce que** le tube est le piston d'une pompe à haute pression.

14. Appareil selon l'une quelconque des revendications précédentes, **caractérisé en ce que** la formulation médicamenteuse liquide à administrer contient une substance avec une pression de vapeur élevée ou un composé alcoolique.

15. Utilisation de l'appareil selon l'une quelconque des revendications précédentes, **caractérisée en ce que** la formulation médicamenteuse liquide est administrée par une buse (12) sur l'appareil en tant qu'aérosol en particulier en tant qu'aérosol inhalable.
